# EUROPEAN PATENT SPECIFICATION

(11) **EP 4 593 897 B1**
(45) Date of publication and mention of the grant of the patent: **24.06.2026**
(21) Application number: 23794141.4
(22) Date of filing: 27.09.2023
(51) Int. Cl.: A61L 2/26, A61B 90/70, F16J 13/02, F16J 13/16, F16J 15/02

(54) **LID ASSEMBLY FOR MEDICAL DEVICE PROCESSOR**
DECKELANORDNUNG FÜR PROZESSOR MEDIZINISCHER VORRICHTUNGEN
ENSEMBLE COUVERCLE POUR UN PROCESSEUR DE DISPOSITIF MÉDICAL

(30) Priority: 30.09.2022 US 202263411662 P; 18.10.2022 US 202263379965 P
(43) Date of publication of application: 06.08.2025
(73) Proprietor: American Sterilizer Company, Mentor, OH 44060 (US)
(72) Inventor: LANGE, Ethan K., Painesville, Ohio 44077 (US); LAMBERT, Sean E., Wickliffe, Ohio 44092 (US); HASSEN, Jacob R., Mentor, Ohio 44060 (US)
(74) Representative: FRKelly
(86) International application number: PCT/US2023/033778
(87) International publication number: WO 2024/072837

(56) References cited:
- CN-A- 105 088 705
- JP-A- 2009 261 513
- US-A1- 2013 098 407

## Description

### Field of Invention

This application relates generally to a lid assembly for a medical device processer, and more particularly to mechanisms for replacing components of the lid assembly for the medical device processor and mechanisms for opening, closing, and sealing the lid assembly for the medical device processor.

### Background

Medical device processors, also referred to as medical instrument processors, are widely used in various health care settings and are typically associated with a sterilization claim or a high level disinfection claim. An example of such a processor is an automated endoscope reprocessor (AER) used for reprocessing endoscopes, such as duodenoscopes, and endoscope accessories. AERs are designed to kill microorganisms in or on reusable endoscopes by exposing their outside surfaces and interior channels to liquid chemical sterilant or high level disinfectant solutions. Some medical device processors include a processing basin with a hinging lid assembly or door. The lid is held open while an endoscope is loaded into the processing basin. Prior to starting the processing cycle, the lid is closed, locked, and sealed. After the cycle is completed, the lid is unsealed, unlocked, and opened so the processed device can be removed from the basin.

Considering that the user is either handling a soiled/dirty endoscope before processing or a clean/sterile endoscope after processing when the lid needs to be opened or closed, it is ideal if the user can avoid touching the lid to avoid any cross-contamination. As a result, it has become commonplace for medical device processors such as AERs to have automated opening and closing lid assemblies. Some processors utilize a pneumatic system to open and close the lid, while others have spring-loaded mechanisms to open the lid when it is unlatched. In addition to the opening and closing operation, the lid assembly must have a means to lock and unlock, and to seal and unseal. In the case of a power failure during a cycle phase where there is peracetic acid or other chemistry in the processing basin, the lid must remain locked until fluid drains from the basin. The seal and unseal functionality allows the processing basin to be a self-contained sealed system while a cycle is completed. This enhances process efficacy by preventing ingress of unsterile air into the processing basin. Additionally, a sealed basin prevents fluid egress from the processing basin.

For medical device processors that have hinging lid assemblies, there remain various shortcomings, drawbacks, and disadvantages relative to certain applications.

For example, the lid assemblies of some medical device processors may also include a seal and clear viewing window. Because the seal and window are exposed to process chemistries during a processing cycle, these components are the most prone to material degradation and may require replacement over the life of the product. Some existing processors require the entire lid assembly including a cast frame, the seal and viewing window, latches, overflow piping, etc. to be replaced if the viewing window cracks or the seal begins to leak. The process of replacing the lid assembly can be difficult to perform by a single technician without assistance, as the assembly may be bulky and/or heavy.

The sealing of the lid assembly requires an applied compression force. Existing systems perform the sealing operation with an additional mechanism, that is, an additional pneumatic cylinder, secondary inflatable seal, etc. that is separate from the opening and/or closing mechanism or require the user to apply the sealing force manually when closing the lid. A sealing system for one processor oftentimes is incompatible for another processor owing in part to the difference in opening and closing mechanisms of the processors. Manually applying the sealing force involves an additional risk of human error.

For systems which automate the sealing operation with a force applied using compressed air, feedback to a controller of the medical device processor on the "sealed" state is typically performed by monitoring the pressure of the compressed air. This system design is not ideal, as it only monitors for problems in the pneumatic system, such as a leak or a valve failure, and does not monitor for seal compression problems due to obstructions or mechanism failures.

Systems which automate the closing operation typically monitor for obstructions that may be pinched between the basin and the lid as it closes. Systems can employ tape switches, light screens, or another sensing method to allow the controller to detect an obstruction and re-open the lid. These obstruction detection methods are viable for sliding door systems because the chamber opening exists on a single plane. However, for a hinging lid, the lid opening covers multiple planes on the front, left, and right sides. As such, the area which needs to be monitored for obstructions can be very large.

Existing systems also either have automatic or manual closing of the lid, but do not have the capability for both.

Accordingly, there remains a need for further contributions in this area of technology.

### Summary of Invention

According to one aspect of the invention, a medical device processor includes a basin, a lid frame, and a seal window. The basin has an opening for receiving therein a medical device for processing and a rim at the perimeter of the opening. The seal window assembly is removably mounted to the lid frame. The seal window assembly includes a window and an elastomeric seal fixed to the window. The lid frame with the seal window assembly mounted thereto is displaceable relative to the rim of the basin between an open position and a sealed position, wherein in the open position the seal window assembly is at a non-zero angle relative to the seal window assembly in the sealed position and the seal window assembly exposes the opening, In the sealed position the elastomeric seal of the seal window assembly is in sealing contact with the rim of the basin to create a seal between the window and the rim to seal the opening of the basin from the exterior of the medical device processor.

Embodiments of the invention may include one or more of the following additional features separately or in combination.

The lid frame includes a rail structure configured for slidably guiding an edge structure of the window of the seal window assembly to removably mount the seal window assembly to the lid frame.

The rail structure may be lined with cushioning strips secured thereto against which a surface of the edge structure of the window slides during sliding movement relative to the rail structure.

The cushioning strips may include one or more of felt strips and fiberglass.

The rail structure may include at least one support rail and the edge structure may include at least one edge.

The rail structure may include left, right, and rear support rails, and the edge structure may include left, right, and rear edges, and the lid frame may further include an open front end that is configured such that the left, right, and rear edges are insertable and removable through the open front end and slidably guided by the respective left, right, and rear support rails.

The rail structure may have a concave structure, and the edge structure of the window may have a convex structure such that the edge structure of the window is slidably movable within the concave structure of the rail structure.

The concave structure may be C shape in cross section and cushioning strips may be secured to an underside of one of the arms of the C shape structure against which a surface of the convex structure of the window slides during sliding movement within the concave structure.

The lid frame may be mounted to the basin for pivotable movement about a hinge axis and the seal window assembly may be removably mounted to the lid frame for slidable movement in a direction perpendicular to the hinge axis.

The medical device processor may further include rear hinge brackets fixed to a rear of the lid frame and pivotably mounted to respective rear actuation assemblies of the medical device processor for pivotable movement about the hinge axis.

The rear hinge brackets may be fixed to an upper surface of the lid frame such that the hinge axis is above the upper surface.

The lid frame may further include an open front end that is configured such that the edge structure of the window is insertable and removable through the open front end and slidably guided by the rail structure.

The medical device processor may further include a cover panel removably mounted to a front support member of the lid frame to support a front edge of the edge structure of the window and prevent sliding movement of the window relative to the rail structure.

The cover panel may include downwardly protruding latch bolts configured to be engaged by respective front actuation assemblies of the medical device processor.

The rim, the lid frame, and the elastomeric seal may each be annular in shape.

The seal window assembly may be configured such that when the lid frame is in the sealed position the window enables viewing of the interior of the basin.

According to another aspect of the invention, a medical device processor includes a basin, a lid, an elastomeric seal, front actuation assemblies, and rear actuation assemblies. The basin has an opening for receiving therein a medical device for processing and a rim at the perimeter of the opening. The front actuation assemblies are at a front of the basin and the rear actuation assemblies are at a rear of the basin. The lid is configured for angular displacement about a hinge axis of the lid between a closed position and an open position, wherein in the closed position the lid covers the opening, and wherein in the open position the lid is at a non-zero angle relative to the lid in the closed position and the lid exposes the opening. The front actuation assemblies and the rear actuation assemblies are configured to displace the lid from the closed position to a sealed position, wherein in the sealed position the lid compresses the elastomeric seal between the lid and the rim of the basin to create a seal between the lid and the rim to seal the opening of the basin from the exterior of the medical device processor.

Embodiments of the invention may include one or more of the following additional features separately or in combination.

The rear actuation assemblies may be configured to angularly displace the lid about the hinge axis of the lid from the closed position to the open position.

The rear actuation assemblies may be configured to angularly displace the lid about the hinge axis of the lid from the closed position to the open position from above the lid.

The lid may be configured to be angularly displaced from the open position to the closed position by gravity.

The elastomeric seal may be fixed to an underside of the lid.

The front actuation assemblies and the rear actuation assemblies may be configured to vertically displace the lid from the closed position to the sealed position.

The front actuation assemblies may be disposed beneath the basin.

The rear actuation assemblies may be disposed above the basin.

The front actuation assemblies may include a latch assembly configured to latch a latch bolt of the lid to lock the lid in the closed position and to release the latch bolt to enable the lid to be displaced from the closed position to the open position.

The medical device processor may further include a housing, and the latch assembly may be pivotably mounted to the housing for pivotable movement between a latched state and a sealed state.

The front actuation assemblies may include a seal actuator configured when actuated to pivot the latch assembly from the latched state wherein the lid is in the closed position to the sealed state wherein the latch assembly displaces the latch bolt downward to urge the lid from the closed position to the sealed position.

The seal actuator may include a pneumatic cylinder.

The pneumatic cylinder may include a single-acting, push style actuator.

The front actuation assemblies may include a latch assembly return spring configured such that when the seal actuator is released the latch assembly return spring urges the latch assembly from the sealed state back to the latched state.

The latch assembly may include a latch pawl configured to latch and release the latch bolt, and the latch pawl may include a latch pawl extension, and the seal actuator may be configured when actuated to exert a force on the latch pawl extension to pivot the latch assembly from the latched state to the sealed state.

The medical device processor may further include means for reducing the friction between the seal actuator and the latch pawl extension.

The latch assembly may include a latch release trigger that when triggered causes the latch pawl to release the latch bolt to enable the lid to be displaced from the closed position to the open position.

The front actuation assemblies may include an unlatch actuator configured when actuated, and with the lid in the closed position, to trigger the latch release trigger.

The medical device processor may further include means for reducing the friction between the unlatch actuator and the latch release trigger.

The unlatch actuator may include a pneumatic cylinder.

The pneumatic cylinder may include a single-acting, push style actuator.

The medical device processor may further include front lid sealed detection switches configured to detect displacement of a front of the lid between the closed position and the sealed position.

The front lid sealed detection switches may include limit switches that are configured to detect when the lid has reached a predetermined limit position corresponding to a predetermined sealing force applied to the elastomeric seal.

The medical device processor may further include a latch bolt detection lever pivotably mounted to the housing and in the path of the latch bolt of the lid such that as the latch bolt is displaced downward the latch bolt rotates the latch bolt detection lever so that the latch bolt detection lever depresses a limit switch to provide feedback to a controller that the lid is in the sealed position.

The latch bolt detection lever may be configured to flex as the latch bolt detection lever depresses the limit switch.

The latch bolt detection lever may be configured such that as the latch bolt is displaced upward the latch bolt detection lever releases the limit switch to provide feedback to the controller that the lid is in the closed position.

The rear actuation assemblies may include a seal lift actuator configured to exert a first force in a first direction to displace the lid from the closed position to the open position and to exert a second force in a second direction that is opposite the first direction to displace the lid from the closed position to the sealed position.

The seal lift actuator may include a double acting pneumatic cylinder.

The medical device processor may further include a housing and a seal lift bar, wherein the seal lift bar pivotably couples the seal lift actuator to the lid and is levered relative to the housing to increase the magnitude of the compressive force on the elastomeric seal.

The seal lift bar may be configured such that, as the seal lift actuator exerts the second force to displace the lid from the closed position to the sealed position, the seal lift bar exerts an upward force against a stop member of the housing to increase the magnitude of the compressive force on the elastomeric seal.

The seal lift bar may be pivotably mounted to the lid about the hinge axis such that, when the second force exerted by the seal lift actuator is released and the lid is in the closed position, the seal lift bar is spring biased to a home position in which the seal lift bar abuts a stop member of the housing.

The seal lift bar may be spring biased to reduce jerk as the seal lift actuator exerts the first force to displace the lid from the closed position to the open position.

The seal lift bar may be spring biased relative to the upper surface of the lid by a seal lift bar return spring beneath the seal lift bar that biases the seal lift bar away from the upper surface and a lift jerk reduction spring on top of the seal lift bar that biases the seal lift bar toward the upper surface.

The seal lift bar may have front and rear ends and an intermediate portion, wherein the lid is pivotably mounted to the intermediate portion about the hinge axis, the seal lift actuator is pivotably mounted to the front end, and the rear end is a free end that abuts an underside of a stop member of the housing.

The seal lift actuator may be pivotably mounted to the seal lift bar at a seal lift pivot located between the hinge axis and the front of the basin, and the seal lift bar may extend rearwardly from the hinge axis to abut an underside of a stop member of the housing.

The medical device processor may be configured such that when viewed from above the lid is pivotably mounted to the intermediate portion about the hinge axis at a location radially outside of the perimeter of the elastomeric seal.

The medical device processor may further include rear lid sealed detection switches configured to detect displacement of a rear of the lid between the closed position and the sealed position.

The rear lid sealed detection switches may include limit switches that are configured to detect when the lid has reached a predetermined limit position corresponding to a predetermined sealing force applied to the elastomeric seal.

The medical device processor may further include a limit switch in the path of the seal lift bar such that as the lid is displaced from the closed position to the sealed position the seal lift bar depresses the limit switch to provide feedback to a controller that the lid is in the sealed position.

The limit switch may be configured such that when the second force exerted by the seal lift actuator is released and the lid is in the closed position, the seal lift bar releases the limit switch to provide feedback to the controller that the lid is in the closed position.

According to another aspect of the invention, a medical device processor includes a basin, a lid, rear actuation assemblies, and a controller. The basin has an opening for receiving therein a medical device for processing. The rear actuation assemblies are at a rear of the basin. The rear actuation assemblies are configured to be pressurized with a lid lift pressure to angularly displace the lid about a hinge axis of the lid from a closed position to an open position, wherein in the closed position the lid covers the opening, and wherein in the open position the lid is at a non-zero angle relative to the lid in the closed position and the lid exposes the opening. The lid is configured to be angularly displaced about the hinge axis from the open position to the closed position. The controller is configured to control pressurization of the rear actuation assemblies and to monitor the lid lift pressure of the rear actuation assemblies as the lid moves from the open position to the closed position. The controller is further configured such that, as the lid moves from the open position toward the closed position, if the lid lift pressure of the rear actuation assemblies reaches or falls below a predetermined pressure P2 prior to the closing of the lid, the controller pressurizes the rear actuation assemblies with a sufficient pressure to stop further movement of the lid or to angularly displace the lid to the open position.

Embodiments of the invention may include one or more of the following additional features separately or in combination.

The lid may be configured to be angularly displaced about the hinge axis from the open position to the closed position by exhausting the lid lift pressure of the rear actuation assemblies.

The rear actuation assemblies may be configured to angularly displace the lid from the closed position to the open position from above the lid.

The lid may be configured to be angularly displaced from the open position to the closed position by gravity.

The medical device processor may further include front actuation assemblies at a front of the basin that are configured to latch the lid in the closed position.

The front actuation assemblies and the rear actuation assemblies may be configured to displace the lid from the closed position to a sealed position, wherein in the sealed position the lid compresses an elastomeric seal between the lid and a rim of the basin to create a seal between the lid and the rim to seal the opening of the basin from the exterior of the medical device processor.

The controller may be configured to control unlatching of the front actuation assemblies to unlatch the lid from the closed position and to monitor the lid lift pressure of the rear actuation assemblies in angularly displacing the lid from the closed position to the open position, and the controller may be configured to unlatch the front lock assemblies when the lid lift pressure of the rear actuation assemblies increases to a predetermined pressure P1 is less than what is required to overcome the weight of the lid.

The medical device processor may further include a pressure switch in communication with the controller and configured with a setpoint P1 at the predetermined pressure P1, and the controller may be configured to unlatch the front actuation assemblies upon the pressure switch indicating the setpoint P1 at the predetermined pressure P1 has been reached.

The controller may be configured to monitor a lid closed switch that indicates whether the lid has closed, and if the controller determines the lid closed switch has not yet indicated the lid has closed before the pressure of the rear actuation assemblies has reached the predetermined pressure P2, the controller pressurizes the rear actuation assemblies with the sufficient pressure to stop further movement of the lid or to angularly displace the lid to the open position.

The predetermined pressure P2 may be a pressure less than what is required for the front actuation assemblies to latch the lid in the closed position.

The medical device processor may further include a pressure switch in communication with the controller and configured with a reset point P2 configured at the predetermined pressure P2, wherein, as the lid moves from the open position toward the closed position, if prior to the closing of the lid the pressure switch indicates to the controller that the lid lift pressure has reached or fallen below the reset point P2 at the predetermined pressure P2, the controller is configured to pressurize the rear actuation assemblies with the sufficient pressure to stop further movement of the lid or to angularly displace the lid to the open position.

The controller may be configured such that if the controller determines the lid closed switch indicates the lid has closed before the pressure switch indicates the reset point P2 configured at the predetermined pressure P2 has been reached, the front actuation assemblies latch the lid in the closed position.

The lid closed switch may be configured such that, after the front actuation assemblies latch the lid in the closed position and the reset point P2 configured at the predetermined pressure P2 is reached, the lid closed switch indicates to the controller that the lid is closed.

The medical device processor may further include an over-pressurization valve configured to exhaust the pressure of the rear actuation assemblies when the pressure exceeds a predetermined pressure P3, thereby enabling the lid to be closed manually.

The rear actuation assemblies may include double acting pneumatic cylinders that, when pressurized, exert a lift force on the lid at a lift force angle relative to the lid, wherein the lift force angle changes as the double acting pneumatic cylinders angularly displace the lid from the closed position to the open position.

The medical device processor may further include a lift force balance spring configured to maintain the lid lift pressure and the corresponding lift force substantially constant over a predetermined range of the angular displacement as the lift force angle changes.

The predetermined range may be from the closed position to a predetermined number of degrees before reaching the open position.

The lift force balance spring may be mounted on-axis on a rod of the double acting pneumatic cylinder.

The medical device processor may further include a lid deceleration spring configured, when the lid has been angularly displaced a predetermined number of degrees before reaching the open position, to increase the lid lift pressure and the corresponding lift force required to reach the open position.

The medical device processor may further include a lid deceleration spring configured, when the lid has been angularly displaced a predetermined number of degrees before reaching the open position, to reduce the speed of the lid.

The lid deceleration spring may be mounted on-axis on a rod of the double acting pneumatic cylinder.

The medical device processor may further include a pressure regulator configured to reduce a source pressure to a predetermined pressure P3 sufficient for the rear actuation assemblies to angularly displace the lid from the closed position to the open position and to hold the lid in the open position against gravity and the lift forces of the lift force balance spring and the lid deceleration spring.

The medical device processor may further include a pressure regulator configured to reduce a source pressure to a predetermined pressure P3 sufficient for the rear actuation assemblies to angularly displace the lid from the closed position to the open position and to hold the lid in the open position.

The pressure regulator may include an over-pressurization vent configured to exhaust the pressure of the rear actuation assemblies when the pressure exceeds the predetermined pressure P3, thereby enabling the lid to be closed manually.

The medical device processor may further include a bidirectional flow control valve configured to independently control flowrate of pressurized air supplied to and exhausted from the rear actuation assemblies to control the respective open and close speeds of the lid.

These and further features will be apparent with reference to the following description and attached drawings which set forth certain illustrative embodiments of the invention.

### Brief Description of the Drawings

The annexed drawings, which are not necessarily to scale, show various aspects of the invention.
Fig. 1 is a perspective view of a medical device processor according to an embodiment of the invention and with some housing structure of the medical device processor omitted for clarity.
Fig. 2 is a right side view of the Fig. 1 medical device processor, showing a lid thereof in an open position.
Fig. 3 is a larger version of the Fig. 1 view and with a frame of the medical device processor omitted for clarity.
Fig. 4 is a perspective view of the Fig. 1 medical device processor, showing the lid thereof in a closed position and with some housing structure of the medical device processor omitted for clarity.
Fig. 5 is a state diagram showing transitional states and sub-phases of the Fig. 1 medical device processor according to an embodiment of the invention.
Fig. 6 is a perspective view of a lid assembly of the Fig. 1 medical device processor according to an embodiment of the invention.
Fig. 7 is bottom perspective of the Fig. 6 lid assembly, showing a lid and cover panel disassembled from a lid frame of the lid assembly.
Fig. 8 is a perspective view of a front actuation assembly of the Fig. 1 medical device processor according to an embodiment of the invention.
Fig. 9 is an exploded perspective view of the Fig. 8 front actuation assembly.
Fig. 10 is a right side view of a lock bracket of the Fig. 8 front actuation assembly.
Fig. 11 is a right side view of a latch assembly of the Fig. 8 front actuation assembly, showing the latch assembly in a latched state.
Fig. 12 is a right side view of the Fig. 11 latch assembly, showing the latch assembly in a sealed state.
Fig. 13 is a right view of the Fig. 11 latch assembly, showing the latch assembly in an unlatched state.
Fig. 14 is a right side view of the Fig. 8 front actuation assembly, showing the front actuation assembly in a latched state.
Fig. 15 is a left side view of the Fig. 8 front actuation assembly, showing the front actuation assembly in the latched state.
Fig. 16 is a right side view of the Fig. 8 front actuation assembly, showing the front actuation assembly in a sealed state.
Fig. 17 is a left side view of the Fig. 8 front actuation assembly, showing the front actuation assembly in the sealed state.
Fig. 18 is a right side view of the Fig. 8 front actuation assembly, showing the front actuation assembly in an unlatched state.
Fig. 19 is a left side view of the Fig. 8 front actuation assembly, showing the front actuation assembly in the unlatched state.
Fig. 20 is a perspective view of a rear actuation assembly of the Fig. 1 medical device processor according to an embodiment of the invention.
Fig. 21 is a larger version of the Fig. 20 view and with a frame of the medical device processor omitted for clarity.
Fig. 22 is an exploded perspective view of the Fig. 20 rear actuation assembly.
Fig. 23 is a larger version of the Fig. 22 and with a frame of the medical device processor omitted for clarity.
Fig. 24 is a partial right side view of the Fig. 20 rear actuation assembly, showing the rear actuation assembly in a closed state.
Fig. 25 is a partial right side view of the Fig. 20 rear actuation assembly, showing the rear actuation assembly in a sealed state with a rear hinge bracket in phantom lines to show a rear lid sealed detection switch.
Fig. 26 is a partial right side view of the Fig. 20 rear actuation assembly, showing the rear actuation assembly in an opening/closing constant volume sub-phase.
Fig. 27 is a partial right side view of the Fig. 20 rear actuation assembly, showing the rear actuation assembly in an opening/closing constant pressure sub-phase.
Fig. 28 is a partial right side view of the Fig. 20 rear actuation assembly, showing the rear actuation assembly in an open state.
Fig. 29 is a line graph showing the relationship between required opening/closing pressure and lid angle where the rear actuation assembly does not have a lift force balance spring.
Fig. 30 is a line graph showing the relationship between required opening/closing pressure and lid angle where the rear actuation assembly has a lift force balance spring.
Fig. 31 is a line graph showing the relationship between required opening/closing pressure and lid angle where the rear actuation assembly does not have a lid deceleration spring.
Fig. 32 is a line graph showing the relationship between required opening/closing pressure and lid angle where the rear actuation assembly has a lid deceleration spring.
Fig. 33 shows a schematic diagram of a pneumatic system and a controller configured to control components of the Fig. 1 medical device processor in accordance with an embodiment of the invention.
Figs. 34A and 34B show a state diagram showing an example of the Fig. 33 controller interfaced with components of the medical device processor in accordance with an embodiment of the invention.
Fig. 35 shows a chart identifying the state of a component identified in the left column during a phase of the medical device processor identified in the top row in accordance with an embodiment of the invention, wherein the components correspond to the components shown in the Fig. 33 schematic diagram and the Figs. 34A and 34B state diagram.
Fig. 36 is a graph showing a lid angle and a lid lift pressure of a lid assembly versus time during an opening phase of the medical device processor in accordance with an embodiment of the invention.
Fig. 37 is a graph showing a lid angle and a lid lift pressure of a lid assembly versus time during a closing phase of the medical device processor in accordance with an embodiment of the invention.

### Detailed Description

While the present invention can take many different forms, for the purpose of promoting an understanding of the principles of the invention, reference will now be made to the embodiments illustrated in the drawings and specific language will be used to describe the same. It will nevertheless be understood that no limitation of the scope of the invention is thereby intended. Any alterations and further modifications of the described embodiments, and any further applications of the principles of the invention as described herein, are contemplated as would normally occur to one skilled in the art to which the invention relates.

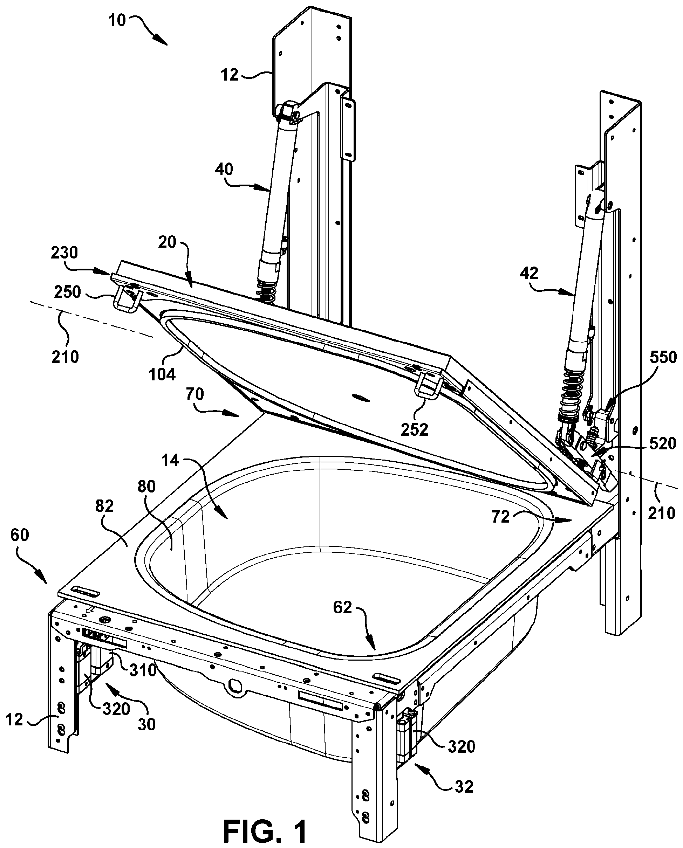

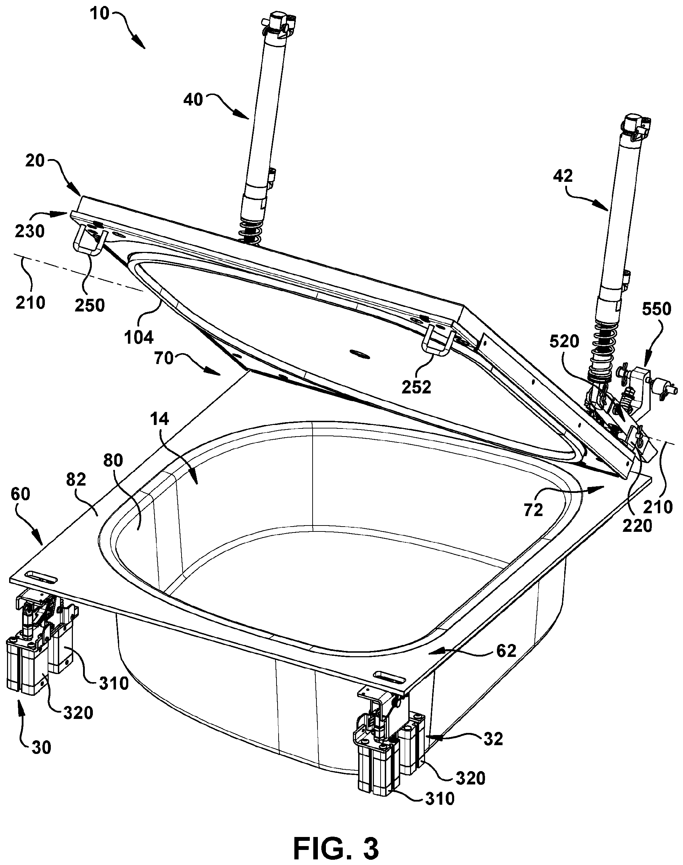

Figs. 1-4 show a medical device processor 10 in accordance with an embodiment of the invention. The medical device processor 10 may be any type of system for decontaminating medical devices or instruments, for example, by a sterilization process or a disinfection process. In the illustrated embodiment, the medical device processor 10 is an automated endoscope reprocessor (AER) used for reprocessing endoscopes, such as duodenoscopes, and endoscope accessories, and configured to kill microorganisms in or on reusable endoscopes by exposing their outside surfaces and interior channels to liquid chemical sterilant or high level disinfectant solutions.

The medical device processor 10 includes a housing or frame 12, a processing basin 14, a lid assembly 20 also referred to herein as a lid, two front actuation assemblies 30, 32, two rear actuation assemblies 40, 42, a pneumatic system 50, and a controller 52, each described in turn below. In some embodiments, there may be more than two front actuation assemblies 30, 32 and/or more than two rear actuation assemblies 40, 42, for example three or four such assemblies where, for example, the medical device processor 10 is relatively wider.

In the illustrative embodiment, the lid assembly 20 is situated above the processing basin 14 such that it opens at the front and hinges at the rear. The two front actuation assemblies 30, 32 are located beneath the processing basin 14 and are mounted to the frame 12 at a front-left corner 60 and a front-right corner 62. The two rear actuation assemblies 40, 42 are located above the lid assembly 20 and thus above the processing basin 14 at a rear-left corner 70 and a rear-right corner 72.

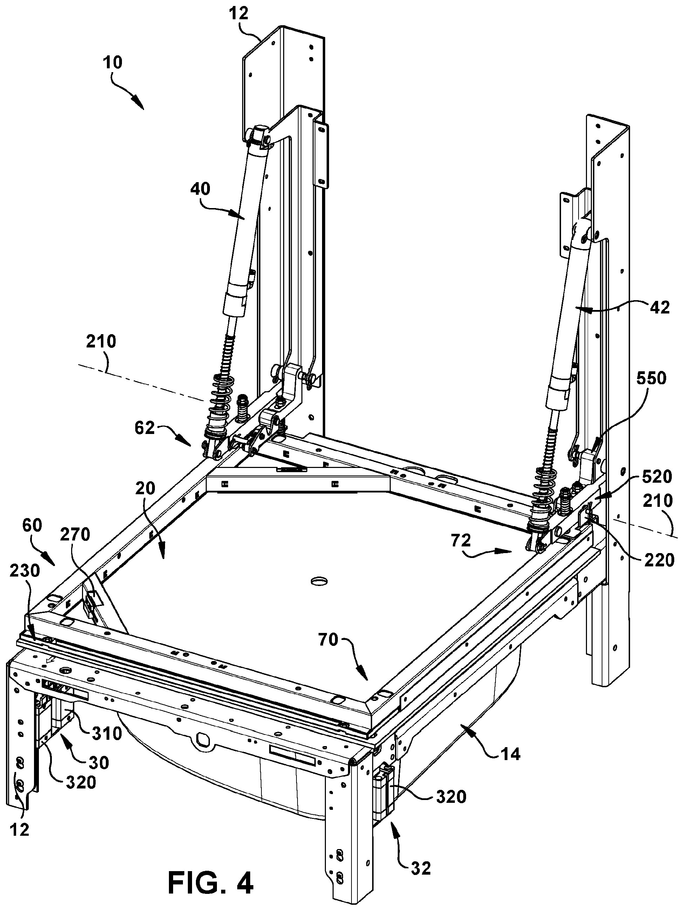

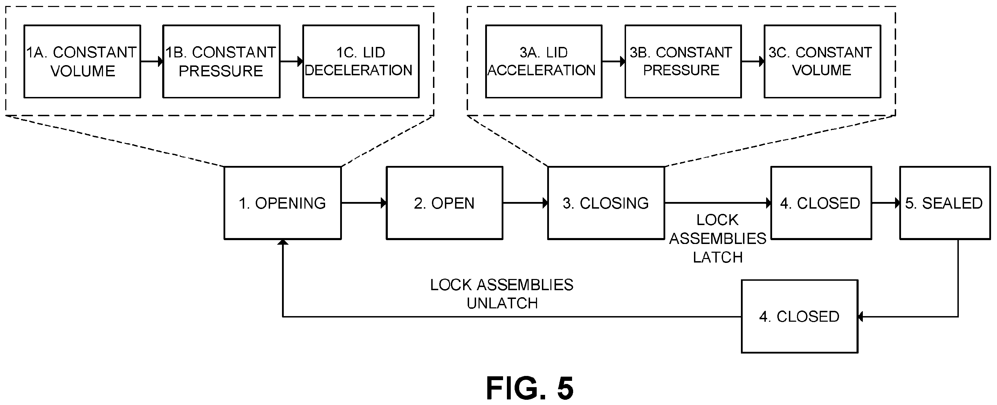

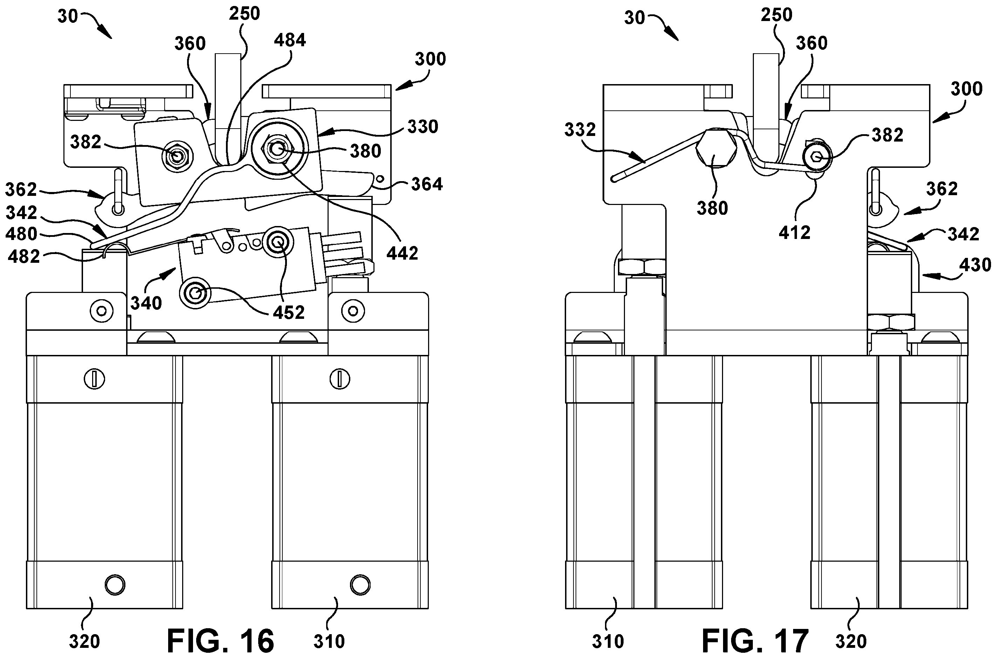

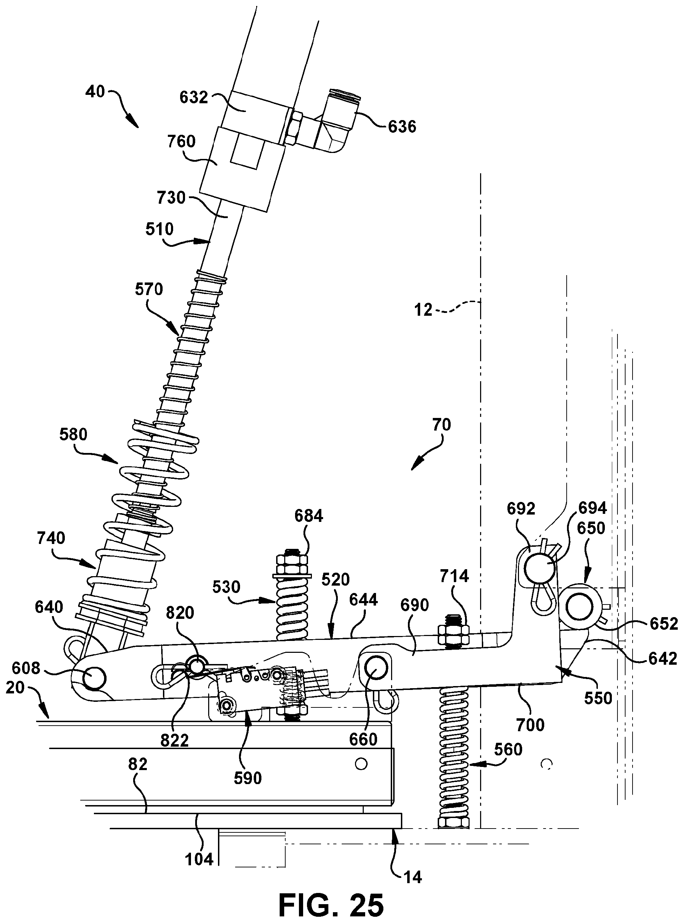

Referring to Fig. 5, the lid assembly 20 has three transitional states, also referred to herein as positions, and several sub-phases which will be described in greater detail below with respect to the different components of the medical device processor 10. Generally, when the lid assembly 20 is in a horizontal orientation, and latched to the front actuation assemblies 30, 32, the lid assembly 20 is latched in a closed state, that is, a closed position, as shown for example in Fig. 4. The horizontal orientation and closed state of the lid assembly 20 is also referred to herein as a home position. Conversely, the lid assembly 20 is in an open state, that is, an open position, when the lid assembly 20 is held at a non-zero angle relative to the position of the lid assembly 20 in the closed state, as shown for example in Figs. 1-3. When the lid assembly 20 is in a horizontal orientation and the front actuation assemblies 30, 32 and the rear actuation assemblies 40, 42 impart a downward force on the lid assembly 20, such that it presses down against the processing basin 14, the lid assembly 20 is in a sealed state, that is, a sealed position, as shown for example in later-described Figs. 16 and 25. In the sealed state, the lid assembly 20 is unable to be unlatched and opened by an operator.

In the illustrated embodiment, in the closed state the lid assembly 20 is generally parallel to horizontal, and in the sealed state the lid assembly 20 is also generally parallel to horizontal but vertically displaced relative to the closed state. As will be appreciated, in some embodiments, the lid assembly 20 may seal the processing basin 14 when in the closed state, such that vertical displacement between the closed state and the sealed state may be omitted; in other words, in the sealed state the lid is also in the closed state. As used herein, horizontal means the plane of the horizon, and vertical means at a right angle, or perpendicular, to the horizontal or horizon.

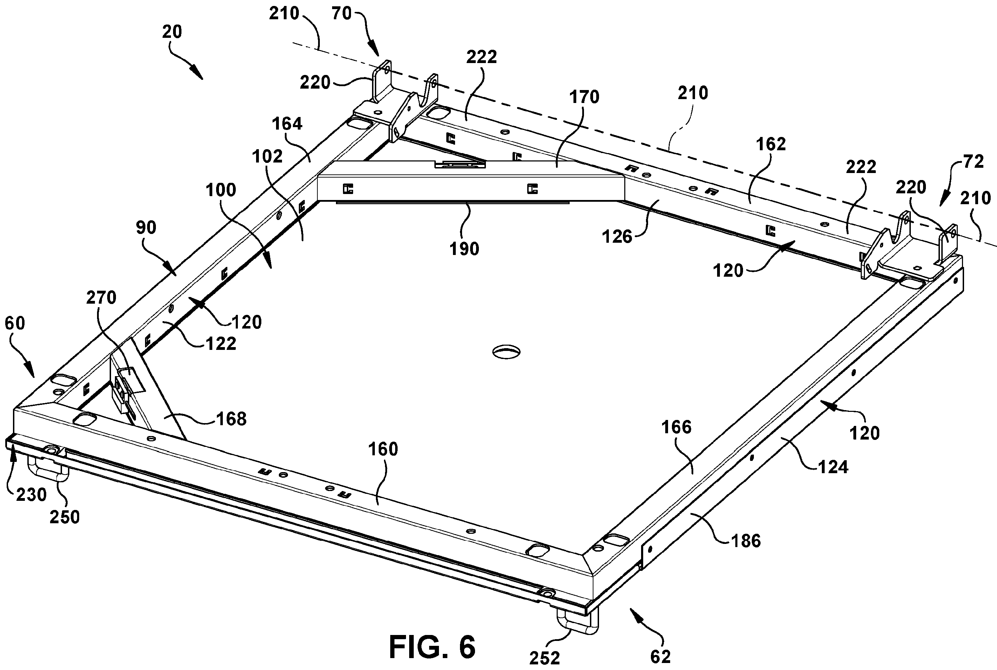

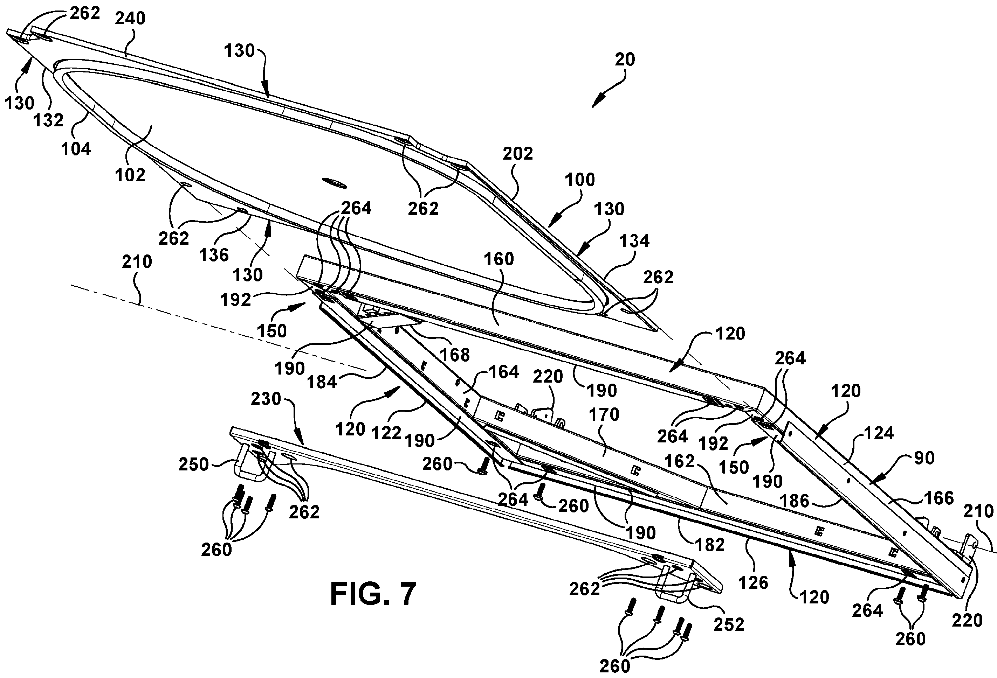

Turning initially then to Figs. 1-4, 6 and 7, an embodiment of the lid assembly 20 of the medical device processor 10 will now be described. As shown in Figs. 1-4, the processing basin 14 of the medical device processor 10 has an opening 80 for receiving therein a medical device for processing and a rim 82 at the perimeter of the opening 80. As shown in Figs. 6 and 7, the lid assembly 20 includes a lid frame 90 and a seal window assembly 100 removably mounted to the lid frame 90. The seal window assembly 100, in turn, is made up of a window 102 and an elastomeric seal 104 fixed to an underside of the window 102, for example by adhesive bonding. In some embodiments, for example as will be described below with respect to the front actuation assemblies 30, 32 and the rear actuation assemblies 40, 42, the lid assembly 20 itself may not include an elastomeric seal 104 and the elastomeric seal 104 may be fixed to the rim 82 of the processing basin 14 rather than to the underside of the window 102. In the illustrated embodiment, the rim 82, the lid frame 90, and the elastomeric seal 104 are each annular in shape. The lid frame 90 with the seal window assembly 100 mounted thereto is displaceable relative to the rim 82 of the basin between the open position shown in Figs. 1-3 and the sealed position shown in Fig. 25. In the open position, the seal window assembly 100 is at a non-zero angle relative to the seal window assembly 100 in the sealed position and the seal window assembly 100 exposes the opening 80. In the sealed position, the elastomeric seal 104 of the seal window assembly 100 is in sealing contact with the rim 82 of the processing basin 14 to create a seal between the window 102 and the rim 82 to seal the opening 80 of the processing basin 14 from the exterior of the medical device processor 10. With the lid frame 90 in the sealed position, the seal window assembly 100 enables viewing of the interior of the processing basin 14.

As shown in Figs. 6 and 7, the lid frame 90 includes a rail structure 120 configured for slidably guiding an edge structure 130 of the window 102 of the seal window assembly 100 to removably mount the seal window assembly 100 to the lid frame 90. The illustrated rail structure 120 includes left, right, and rear support rails 122, 124, 126, and the edge structure 130 includes left, right, and rear edges 132, 134, 136. In some embodiments, the rail structure 120 may include only one support rail, for example a round support rail, and the edge structure 130 may include only one edge, for example a round edge. The lid frame 90 may further include an open front end 150 that is configured such that the edge structure 130 of the window 102 is insertable and removable through the open front end 150 and slidably guided by the rail structure 120. In the illustrated lid assembly 20, the open front end 150 is configured such that the left, right, and rear edges 132, 134, 136 of the window 102 are insertable and removable through the open front end 150 and slidably guided by the respective left, right, and rear support rails 122, 124, 126. Also in the illustrated embodiment, the rail structure 120 has a concave structure, and the edge structure 130 of the window 102 has a convex structure such that the edge structure 130 of the window 102 is slidably movable within the concave structure of the rail structure 120. In an alternate embodiment, the rail structure 120 may have a convex structure and the edge structure 130 may have a concave structure such that the edge structure 130 is slidably movable on the rail structure 120.

The afore described lid frame 90 may be formed by any suitable manufacturing means. In the illustrative embodiment, the lid frame 90 is formed by a weldment having six square metal tubing members, that is, a front support member 160, a rear support member 162, a left support member 164, a right support member 166, a front gusset 168, and a rear gusset 170. The weldment also includes three L-shape lid support rails 182, 184, 186 on the rear, left and right support members 162, 164, 166.

The rail structure 120 is lined with cushioning strips 190 secured thereto against which a surface 202 of the edge structure 130 of the window 102 slides during sliding movement relative to the rail structure 120. The cushioning strips 190 enable the window 102 to slide more easily when removably mounting the seal window assembly 100 to the lid frame 90. Moreover, during a sealing operation the cushioning strips 190 provide some load balancing between the lid frame 90 and the seal window assembly 100 so that the loading between the lid frame 90 and the seal window assembly 100 is more evenly distributed. The cushioning strips 190 may include for example felt strips and/or fiberglass, or other low friction cushioning elements or materials. The cushioning strips 190 may be secured to the rail structure 120 by an adhesive bond or any other suitable securing method. In the illustrative embodiment, the concave structure of the rail structure 120 has a C-shape in cross section and the cushioning strips 190 are secured to an underside 192 of one of the arms of the C-shape structure such that the surface 202 of the convex structure of the window 102 slides against the cushioning strips 190 during sliding movement within the concave structure.

Referring again to Figs. 1-4, 6 and 7, the lid assembly 20 and more specifically the lid frame 90 thereof is mounted to the processing basin 14 for pivotable movement about a hinge axis 210. Rear hinge brackets 220 having for example a U-shape structure are fixed to the lid frame 90, in the illustrated embodiment at the rear-left corner 70 and the rear-right corner 72 thereof. As will be described in greater detail below, the rear hinge brackets 220 are pivotably mounted to the respective rear actuation assemblies 40, 42 of the medical device processor 10 such that the rear actuation assemblies 40, 42 angularly displace the lid assembly 20 about the hinge axis 210. As shown in Figs. 1-4 and 6, the rear hinge brackets 220 are fixed to an upper surface 222 of the lid frame 90 such that the hinge axis 210 is above the upper surface 222. This provides a unique form factor and aids in the medical device processor 10 being narrower than prior processors since the opening and closing of the lid assembly 20 is from above the processing basin 14 rather than, for example, from the sides or rear of the processing basin 14.

With continued reference to Fig. 7, the seal window assembly 100 is removably mounted to the lid frame 90 for slidable movement in a direction perpendicular to the hinge axis 210. As will be appreciated, the lid assembly 20 may be configured so that the slidable movement, or direction of insertion and removal, of the seal window assembly 100 relative to the lid frame 90 is via the left side or the right side, that is, parallel to the hinge axis 210. This can be accomplished for example by changing the location of the open end, wherein instead of having an open front end 150, an open left end is provided that opens to the left or an open right end is provided that opens to the right. Additionally, or alternately, the location of the support rails that make up the rail structure 120 may be changed. For example, instead of left, right and rear support rails 122, 124, 126, the lid frame 90 may instead comprise front, right and rear support rails and open to the left, or comprise front, left and rear support rails and open to the right.

The lid assembly 20 of the medical device processor 10 also includes a cover panel 230 that is removably mounted to the front support member 160 of the lid frame 90. The cover panel 230 supports, or holds up, a front edge 240 of the edge structure 130 of the window 102 to prevent sliding movement of the window 102 from the rail structure 120 of the lid frame 90. The cover panel 230 includes downwardly protruding latch bolts 250, 252 having for example a U-shape and that function as striker bolts to be engaged by the respective front actuation assemblies 30, 32 of the medical device processor 10, as will be described in greater detail below. The seal window assembly 100 and the cover panel 230 are mounted to the lid frame 90 by fasteners 260 that pass through through-holes 262 in the edge structure 130 of the seal window assembly 100 and the cover panel 230 and thread into threaded openings 264 in the lid frame 90, or into cage nuts latched into the lid frame 90.

The illustrated lid assembly 20 also includes a lid closed switch 270 fastened to the front gusset 168 of the lid frame 90. The lid closed switch 270 may be any type of switch configured to indicate whether the lid assembly 20 is closed. For example, the lid closed switch 270 may comprise a reed switch that detects the presence or absence of a magnet located beneath the processing basin 14. Thus, when the lid assembly 20 is in a closed state, the lid closed switch 270 detects the magnetic field and provides an input to the controller 52 indicating that the lid assembly 20 is closed. Conversely, when the lid assembly 20 is in an open state, the lid closed switch 270 cannot detect the magnetic field because the distance from the magnet is too great; as such, the lid closed switch 270 indicates to the controller 52 that the lid assembly 20 is open.

The removability of the seal window assembly 100 from the lid frame 90 provides several advantages over lid assemblies of prior medical device processors. For example, in the case where the window 102 or elastomeric seal 104 needs to be replaced, having the seal window assembly 100 be removable from the rest of the lid assembly 20 is beneficial because replacement cost and time is reduced. Additionally, the removability of the seal window assembly 100, for example by sliding as described above, simplifies the replacement process and can be performed by a single technician without assistance.

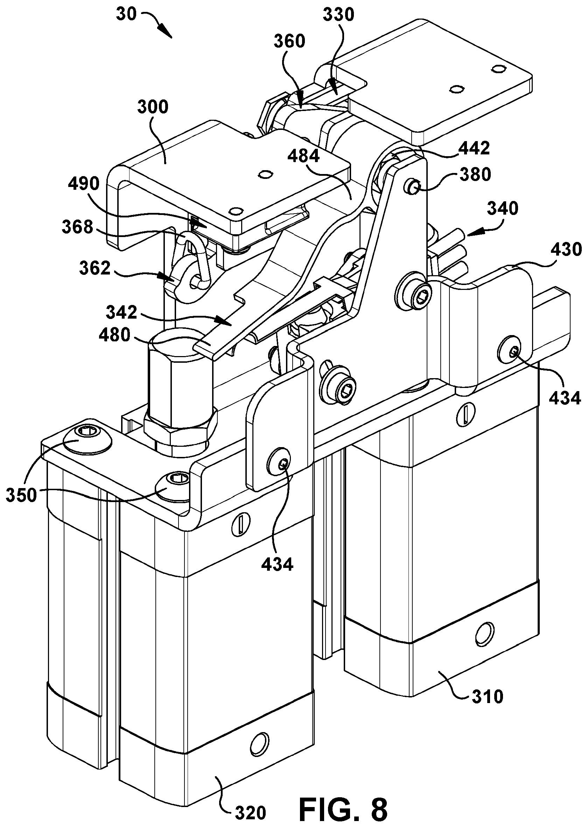

Turning now to Figs. 8-19, the front actuation assemblies 30, 32 according to an embodiment of the invention will now be described. For brevity, in the following, only the front actuation assembly 30 located at the front-left corner 60 of the medical device processor 10 will be described, as such description is equally applicable to the front actuation assembly 32 located at the front-right corner 62 of the medical device processor 10, since in the illustrated embodiment the front actuation assembly 32 is the same as the front actuation assembly 30 except rotated 180 degrees. In some embodiments, the front actuation assemblies 30, 32 may be different in structure, configuration and/or in a non-180 degree relative orientation. Further, in the following the lid assembly 20 may include the elastomeric seal 104 as illustrated, or the lid assembly 20 may not include the elastomeric seal 104 and the elastomeric seal 104 may instead be fixed to the rim 82 of the processing basin 14. Thus, in the description that follows, it is contemplated that the elastomeric seal 104 may be fixed to either the underside of the window 102 or fixed to the rim 82 of the processing basin 14.

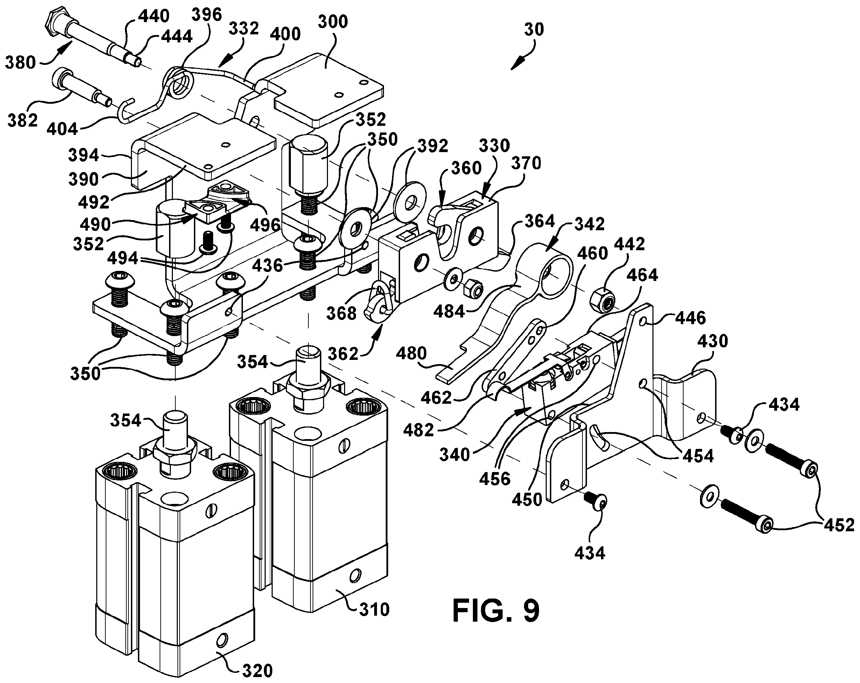

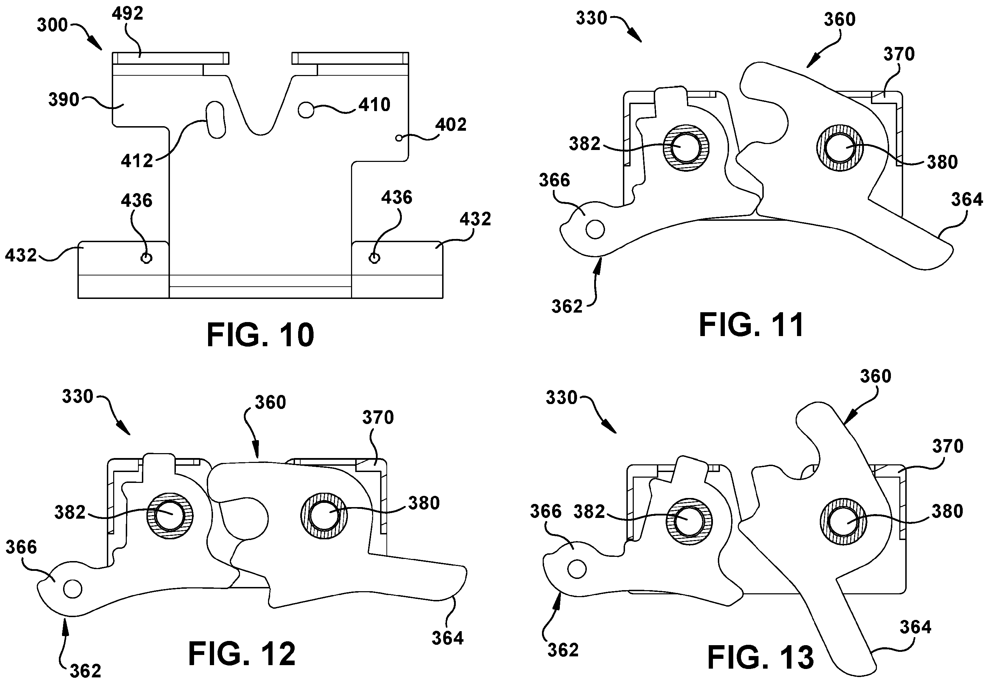

As shown in Figs. 8 and 9, the front actuation assembly 30 includes a lock bracket 300, a seal actuator 310, an unlatch actuator 320, a latch assembly 330, a latch assembly return spring 332, a front lid sealed detection switch 340, and a latch bolt detection lever 342. The lock bracket 300, which is also shown in Fig. 10, has an open C-shape and is fixed to the housing or frame 12 of the medical device processor 10. The seal actuator 310 and the unlatch actuator 320 are mounted to the lower member of the C-shape lock bracket 300 by fasteners 350. The illustrative seal actuator 310 and unlatch actuator 320 are single-acting, push-style, pneumatic cylinder type actuators. In some embodiments, the seal actuator 310 and/or the unlatch actuator 320 may be a double-acting and/or a pull-style and/or a hydraulic or electrical type actuator. The seal actuator 310, when pressurized, exerts a counterclockwise moment of force on a latch pawl 360 of the latch assembly 330. The unlatch actuator 320, when pressurized, exerts a clockwise moment of force on a latch release trigger 362 of the latch assembly 330.

As shown in Fig. 9, a low friction polymeric block 352 may be attached to the actuator rods 354 of the seal actuator 310 and the unlatch actuator 320 to reduce friction and wear when the seal actuator 310 and the unlatch actuator 320 exert an upward force on the respective latch pawl 360 and latch release trigger 362 of the latch assembly 330. In some embodiments, the actuator rods 354 of the seal actuator 310 and the unlatch actuator 320, or blocks 352 attached to the actuator rods 354, may have rollers that rollably contact and thus reduce friction relative to the respective latch pawl 360 and latch release trigger 362. In still other embodiments, the actuator rods 354 may be coupled to the latch pawl 360 and latch release trigger 362 by a lever, a cam, or other suitable connecting member to reduce friction therebetween.

The latch assembly 330 may include any suitable configuration of a latch pawl and latch release trigger operable to latch and release a latch bolt or striker bolt of a lid or door. Exemplary contents of the illustrative latch assembly 330 are shown in Figs. 11-13. The latch pawl 360 and the latch release trigger 362 are mounted within a latch assembly housing 370 on, respectively, a pivot bolt 380 and a slide bolt 382. The latch pawl 360 is torsion spring loaded, or otherwise biased, clockwise in Figs. 11-13, and the latch release trigger 362 is torsion spring loaded, or otherwise biased, counterclockwise in Figs. 11-13. The latch pawl 360 with the latch release trigger 362 engaged therewith, see Fig. 11, holds down the latch bolt 250 of the lid assembly 20 when the lid assembly 20 is in a closed state, see Fig. 5, effectively locking the lid assembly 20. When an upward force is exerted on a latch release extension 366 of the latch release trigger 362, either from the unlatch actuator 320 or via a remote release cable (not shown) attached to the latch release extension 366 by a latch release ring 368, the latch release trigger 362 rotates clockwise to release the latch pawl 360. Referring to Fig. 13, the latch pawl 360, in turn, rotates clockwise to release the latch bolt 250 of the lid assembly 20, enabling the lid assembly 20 to be opened. As will be appreciated, the invention is not intended to be limited to the illustrated latch assembly 330, and other configurations are contemplated. For example, the latch assembly 330 may be configured as a two stage rather than a single stage assembly, and/or may employ other types and configurations of pawls and release triggers for engagement and release of a latch bolt.

As shown in Fig. 9, the latch assembly 330 is set against a right side surface 390 of the lock bracket 300 with low friction washers 392 therebetween to enable relative slidable movement. The latch assembly return spring 332 is a torsion spring located on a left side surface 394 of the lock bracket 300. The latch assembly 330 is mounted to the lock bracket 300 by the pivot bolt 380 and the slide bolt 382. The pivot bolt 380, in the form of a stepped shoulder bolt in the illustrated embodiment, accommodates a main spiral body portion 396 of the latch assembly return spring 332 on the left side surface 394 of the lock bracket 300 and operates as the fixed pivot about which the latch assembly 330 and the latch bolt detection lever 342 rotate on the right side surface 390 of the lock bracket 300. A stationary arm 400 of the latch assembly return spring 332 is received by and held in an aperture 402, see Fig. 10, of the lock bracket 300. The slide bolt 382, in the form of a shoulder bolt in the illustrated embodiment, connects to a mobile arm 404 of the latch assembly return spring 332 on the left side surface 394 of the lock bracket 300 and serves as the sliding pivot for the latch assembly 330 on the right side surface 390 of the lock bracket 300. The pivot bolt 380 is mounted through an opening 410 and serves as a pivot about which the latch assembly 330 and the latch bolt detection lever 342 rotate. The slide bolt 382 is configured to slide within an arc-shape slot 412 in the lock bracket 300.

Referring to Figs. 11-13, the latch assembly return spring 332 spring biases the latch assembly 330 clockwise about the pivot bolt 380. When the seal actuator 310 exerts an upward force on a latch pawl extension 364 of the latch pawl 360, the latch pawl 360 rotates counterclockwise, see Fig. 12, to pull down on the latch bolt 250 of the lid assembly 20. Additionally, as the latch assembly 330 is pivotably mounted to the fixed pivot bolt 380, the full latch assembly 330 rotates counterclockwise against the opposing clockwise moment of force of the mobile arm 404 of the latch assembly return spring 332. This causes the latch pawl 360 to pull the latch bolt 250 downwards an additional distance before bottoming out, ensuring the seal window assembly 100 of the lid assembly 20 makes a complete seal with the rim 82 of the processing basin 14. The latch assembly return spring 332 is pre-loaded such when the seal actuator 310 is released, the mobile arm 404 of the latch assembly return spring 332 returns the latch assembly 330 to a horizontal position.

Referring again to Fig. 9, the front actuation assembly 30 also includes a limit switch bracket 430 mounted to a pair of flanges 432 of the lock bracket 300 by fasteners 434 threaded into threaded holes 436 in the lock bracket 300. The pivot bolt 380 has a threaded portion 440 that threads into a hex nut 442 to axially retain the latch assembly 330 and the latch bolt detection lever 342 in the right-to-left direction while maintaining rotational freedom, and a rounded distal end 444 that is accommodated in a through hole 446 of the limit switch bracket 430 to support the distal end 444 of the pivot bolt 380; that is, so that the pivot bolt 380 is not cantilevered. The front lid sealed detection switch 340 is mounted to a left side surface 450 of the limit switch bracket 430 by fasteners 452 that pass through through-openings 454 in the limit switch bracket 430 and through-openings 456 in the front lid sealed detection switch 340, and thread into respective threaded openings 460 (only one in view in Fig. 9) in a clamp bar 462 on a left side surface 464 of the front lid sealed detection switch 340.

The latch bolt detection lever 342 is mounted on the pivot bolt 380 in a free-rotating manner. The distal end 480 of the latch bolt detection lever 342 is vertically above and in abutting contact with a distal end 482 of the front lid sealed detection switch 340. An intermediate portion 484 of the latch bolt detection lever 342 is vertically below and in the path of travel of the latch bolt 250. As such, when the seal actuator 310 is actuated to urge the latch bolt 250 downward, the latch bolt 250 presses down on the intermediate portion 484 of the latch bolt detection lever 342, which in turn presses down on the front lid sealed detection switch 340. As will be described in greater detail below, the front lid sealed detection switch 340 is configured such that, when depressed by the latch bolt detection lever 342, the front lid sealed detection switch 340 provides an input to the controller 52 indicating that the lid assembly 20 is in a sealed state.

As will be appreciated, the illustrative front lid sealed detection switch 340 in cooperation with the latch bolt detection lever 342 magnify the movement of the latch bolt 250, making adjustment of the front lid sealed detection switch 340 more repeatable. In the illustrative embodiment, the latch bolt detection lever 342 is made of a polymeric or other material to enable the latch bolt detection lever 342 to flex as it depresses the front lid sealed detection switch 340 so that the latch bolt detection lever 342 does not transfer a significant load to the front lid sealed detection switch 340 for example in the case where the latch bolt detection lever 342 bottoms out on the front lid sealed detection switch 340. Such flexibility eliminates any risk of the front lid sealed detection switch 340 adjustment being compromised. One of the through-openings 454 in the limit switch bracket 430 is in the form of an arc-shape slot as shown, enabling the mounting angle of the front lid sealed detection switch 340 to be adjusted on the limit switch bracket 430 such that the front lid sealed detection switch 340 activates at a specific deflection of the latch bolt detection lever 342. In the illustrated embodiment, the front lid sealed detection switch 340 includes a limit switch that is configured to detect when the lid assembly 20 has reached a predetermined limit position corresponding to a predetermined sealing force applied to the elastomeric seal 104.

The front actuation assembly 30 may also include an unlatch cable guide 490 configured to route the remote release cable to the aforementioned latch release ring 368. The illustrated unlatch cable guide 490 is in the form of a polymeric cable redirector block mounted to an upper flange 492 of the lock bracket 300 by fasteners 494 and defining a track 496 that redirects the cable to the latch release ring 368.

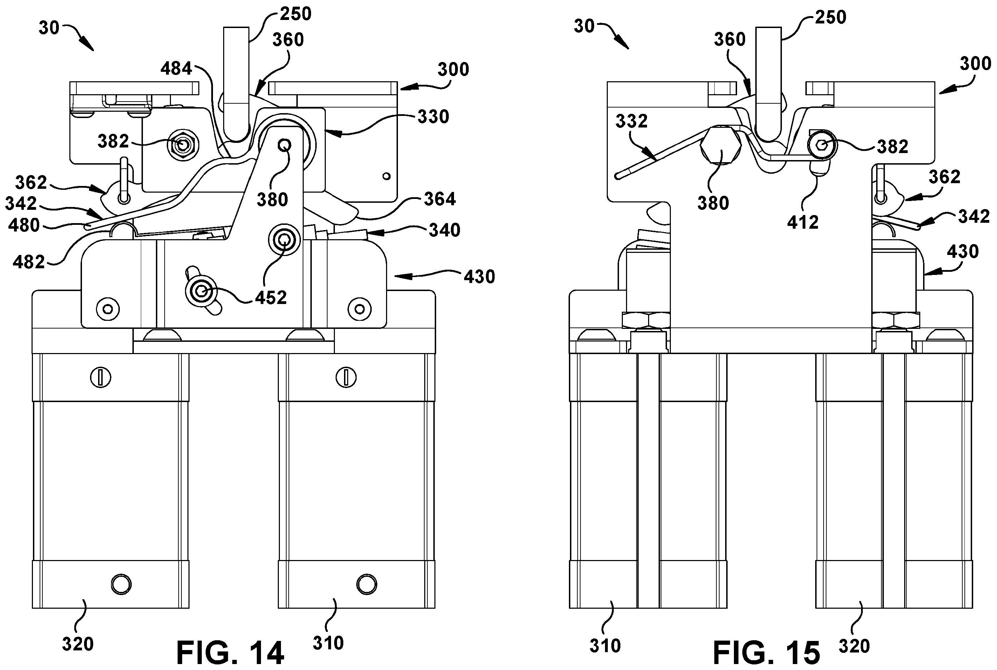

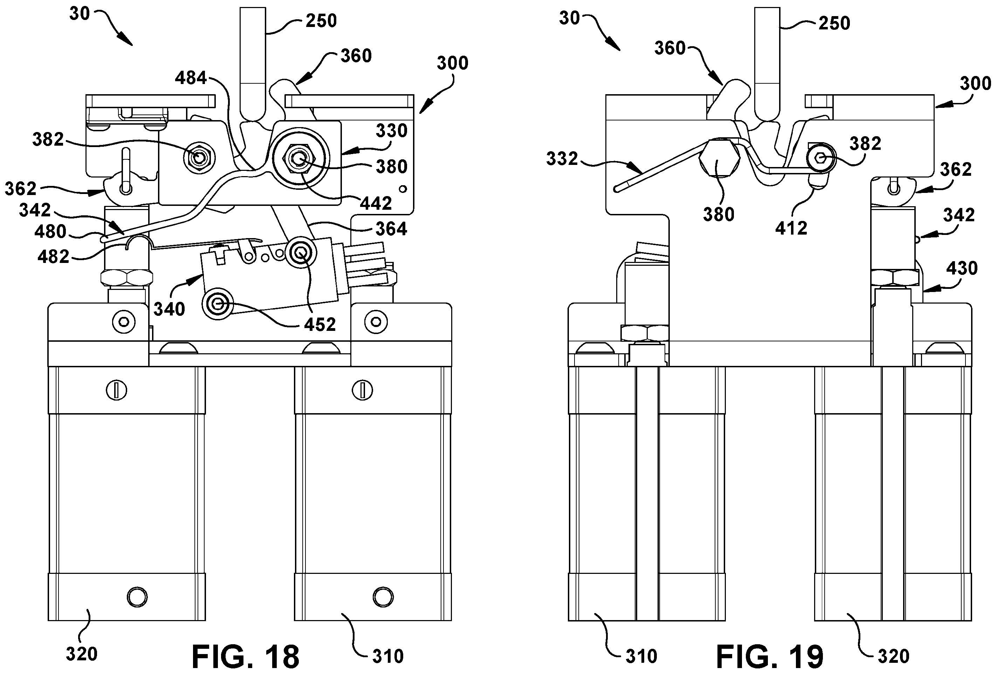

Turning again to the Fig. 5 state diagram, the front actuation assemblies 30, 32 are configured to operate in a latched state as shown Figs. 14 and 15, a sealed state as shown in Figs. 16 and 17, and an unlatched state as shown Figs. 18 and 19. Referring initially to Figs. 14 and 15, the latched state coincides with the lid assembly 20 being in a closed state. In the illustrated embodiment, in the latched state the lid assembly 20 is in the horizontal orientation, or home position, and the latch bolt 250 is retained in the latch assembly 330, thereby preventing the lid assembly 20 from being opened.

The sealed state of the front actuation assemblies 30, 32 coincides with the lid assembly 20 also being sealed. As shown in Figs. 16 and 17, when the seal actuator 310 is pressurized, the seal actuator 310 exerts an upward force on the latch pawl extension 364. This rotates both the latch pawl 360 and the latch assembly 330 counterclockwise about the pivot bolt 380, urging the slide bolt 382 along the arc-shape slot 412 and downward against the opposing force of the latch assembly return spring 332 and the elastomeric seal 104. In so doing, the latch pawl 360 pulls downward on the latch bolt 250 of the lid assembly 20. Thus, owing to the compressive force of the seal actuator 310 being greater than the opposing force of the latch assembly return spring 332 and the compression resistance of the elastomeric seal 104, the front actuation assemblies 30, 32 vertically displace the lid assembly 20 from the closed state to the sealed state. Also, as the latch bolt 250 moves downward, the latch bolt 250 contacts the latch bolt detection lever 342, which in turn, depresses the front lid sealed detection switch 340, indicating to the controller 52 that the lid assembly 20 at the corresponding front actuation assembly 30, 32 is sealed. To unseal the front actuation assemblies 30, 32, the seal actuator 310 is exhausted, which causes the latch assembly return spring 332 and the elastomeric seal 104 to rotate the latch assembly 330 clockwise about the pivot bolt 380 to return the latch assembly 330 to its horizontal position and to thereby revert the lid assembly 20 to the closed state shown in Figs. 14 and 15.

To begin the opening lid assembly 20 transitional phase, see Fig. 5, the lid assembly 20 must be unlatched. Referring to Figs. 18 and 19, when the unlatch actuator 320 is pressurized, the unlatch actuator 320 exerts an upward force on the latch release trigger 362, thereby disengaging or releasing the latch pawl 360. The latch pawl 360 in turn, being spring biased in the clockwise direction, rotates clockwise to release the latch bolt 250, thereby enabling the lid assembly 20 to be opened. Alternatively, the latch release trigger 362 could be pulled upwards using the remote release cable routed through the unlatch cable guide 490 and attached to the latch release ring 368. After the latch bolt 250 is released, the unlatch actuator 320 or the cable are then exhausted/released. The latch release trigger 362 in turn, being spring biased in the counterclockwise direction, rotates counterclockwise to return to its home position, which is a ready state to engage the latch pawl 360 the next time the lid assembly 20 is closed. During the closing lid assembly 20 transition phase, see Fig. 5, the front actuation assemblies 30, 32 return to the latched state shown in Figs. 14 and 15 when the latch bolts 250, 252 press down on the latch pawls 260 by gravity and/or by an operator pushing down on the lid assembly 20. The latch pawls 260 rotate counterclockwise, making sliding cammed contact with the latch release trigger 362, and thereby urging the latch release trigger 362 clockwise until the latch release trigger 362 engages the latch pawl 360 to hold the latch pawl 362 in the latched state, as shown in Fig. 11.

In the above, the latch assembly return spring 332 is a torsion spring that spring biases the latch assembly 330 clockwise about the pivot bolt 380. As will be appreciated, any type of biasing member may be used to spring bias the latch assembly 330 about the pivot bolt 380, and other types are contemplated. For example, a compression spring or a tension spring may be used to spring bias the latch assembly 330 about the pivot bolt 380.

Also, as mentioned above, the illustrated latch assembly 330 is mounted for pivoting functionality. It will be appreciated that the latch assembly 330 may be rigidly mounted to the frame 12 of the medical device processor 10, for example, in applications where the latch assembly 330 latch/lock mechanism and the profile of the elastomeric seal 104 provides a satisfactory seal compression to seal the opening 80 of the processing basin 14 from the exterior of the medical device processor 10.

Also, as mentioned above, the seal actuator 310 and the unlatch actuator 320 are push-type actuators. It will be appreciated that in some embodiments pull-type actuators may be used, for example, where a different configuration of latch assembly 330 is used and/or where gears and/or levers are employed to transmit the actuation force to the components of the particular latch assembly.

As was noted above, the latch bolt detection lever 342 in the illustrated embodiment is configured to flex as it depresses the front lid sealed detection switch 340. In some embodiments, this flexibility aspect of the latch bolt detection lever 342 may be omitted. The lack of flexibility may potentially transfer more load to the front lid sealed detection switch 340, but may still be viable, for example, with a different switch adjustment scheme. In still other embodiments, the latch bolt detection lever 342 itself may be omitted and the front lid sealed detection switch 340 may be configured to detect the latch bolt 250 directly. Omitting the latch bolt detection lever 342 would reduce the number of components but may also reduce the margin for error in switch adjustment.

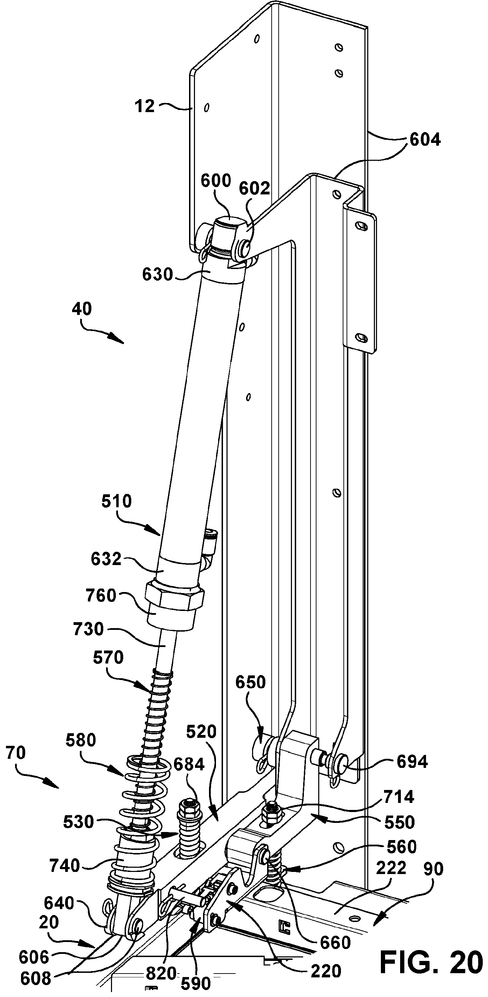

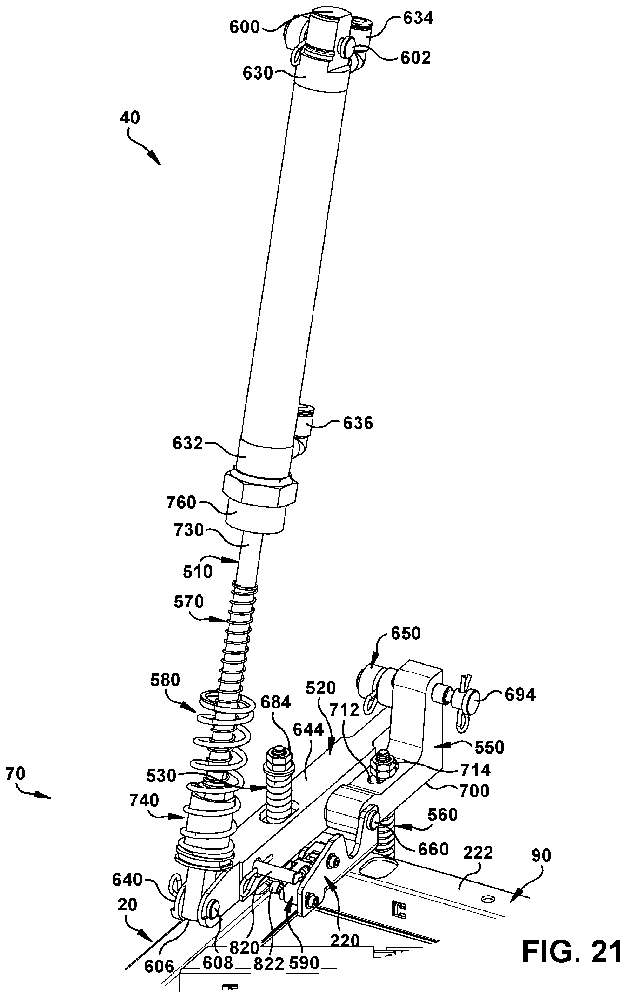

Turning now to Figs. 20-32, the rear actuation assemblies 40, 42 according to an embodiment of the invention will now be described. For brevity, in the following, only the rear actuation assembly 40 located at the rear-left corner 70 of the medical device processor 10 will be described, as such description is equally applicable to the rear actuation assembly 42 located at the rear-right corner 72 of the medical device processor 10, since in the illustrated embodiment the rear actuation assembly 42 is a mirror image of the rear actuation assembly 40. In some embodiments, the rear actuation assemblies 40, 42 may be different in structure, configuration and/or in a non-mirror image relative orientation. Further, in the following the lid assembly 20 may include the elastomeric seal 104 as illustrated, or the lid assembly 20 may not include the elastomeric seal 104 and the elastomeric seal 104 may instead be fixed to the rim 82 of the processing basin 14. Thus, in the description that follows, it is contemplated that the elastomeric seal 104 may be fixed to either the underside of the window 102 or fixed to the rim 82 of the processing basin 14.

As shown in Figs. 20-24, the rear actuation assembly 40 includes a seal lift actuator 510, a seal lift bar 520, a lift jerk reduction spring 530, a seal lift bar return spring 540, a hinge bar 550, a hinge bar return spring 560, a lift force balance spring 570, a lid deceleration spring 580, and a rear lid sealed detection switch 590. The seal lift actuator 510 is located above the lid assembly 20. An upper end 600 of the seal lift actuator 510 is pivotably mounted via a free-rotating pin 602 to a rear frame upright 604 of the medical device processor 10. A lower end 606 of the seal lift actuator 510 is pivotably mounted to the seal lift bar 520 via a free-rotating pin 608. The illustrative seal lift actuator 510 is a double-acting pneumatic cylinder type actuator having a piston side or upper chamber 630 and a rod side or lower chamber 632. Supply/exhaust ports 634, 636 put the respective upper and lower chambers 630, 632 in fluid communication with the pneumatic system 50. In some embodiments, the seal lift actuator 510 may comprise a pair of single-acting actuators and/or a hydraulic or electrical type actuator. When the upper chamber 630 of the seal lift actuator 510 is pressurized, the seal lift actuator 510 exerts a downward force on the seal lift bar 520. Conversely, when the lower chamber 632 of the seal lift actuator 510 is pressurized, the seal lift actuator 510 exerts an upward force on the seal lift bar 520.

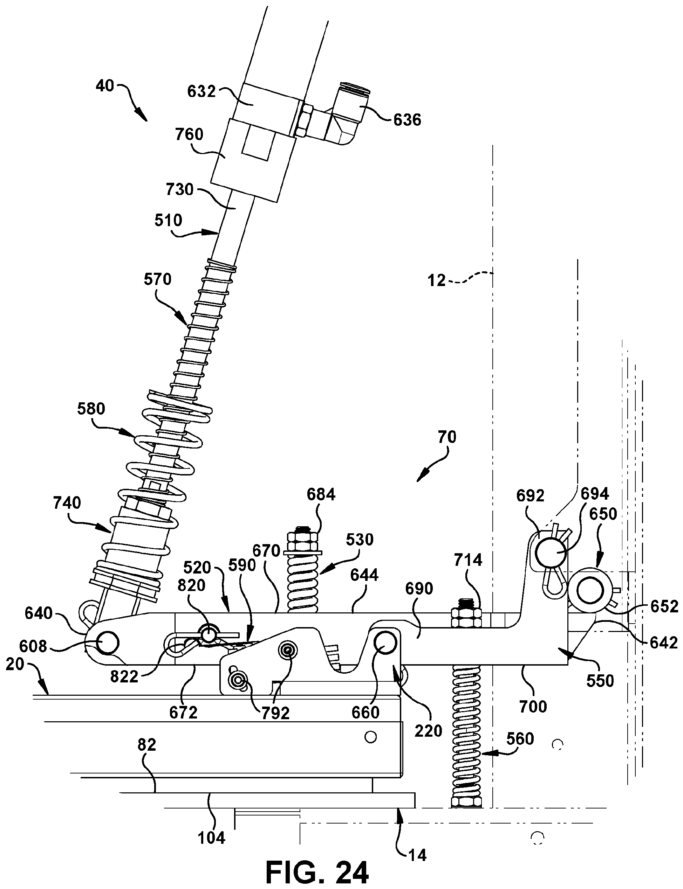

The seal lift bar 520 has a front end 640, a rear end 642, and an intermediate portion 644. The front end 640 is pivotably coupled to the seal lift actuator 510 via the free-rotating pin 608. The rear end 642 is a free end that abuts an underside 652 of a stop member 650 of the rear frame upright 604 when the seal lift bar 520 is in a home position, which in the illustrated embodiment is a horizontal position. The stop member 650 may be for example a polymeric roller mounted to a not shown free-rotating pin. The intermediate portion 644 of the seal lift bar 510 is pivotably mounted to the rear hinge bracket 220 of the lid assembly 20 via a free-rotating pin 660. The center axis of the free-rotating pin 660 thus coincides with the afore described hinge axis 210 about which the lid assembly 20 pivots. As shown in the Figs. 24 and 25 right side views, when viewed from above, the lid assembly 20 is pivotably mounted to the intermediate portion 644 about the hinge axis 210 at a location radially outside of the perimeter of the elastomeric seal 104. As will be appreciated, the stop member 650 provides a leverage point for the seal lift bar 520 such that when the seal lift bar 520 rotates counterclockwise about the free-rotating pin 660, the magnitude of the downward force exerted by the seal lift actuator 510 on the lid assembly 20 increases, resulting in a corresponding increase in the compressive force on the elastomeric seal 104 against the rim 82 of the processing basin 14.

The seal lift bar 520 is spring biased to its home position by the lift jerk reduction spring 530 above, or on a top 670 of, the seal lift bar 520, and by the seal lift bar return spring 540 beneath, or at a bottom 672 of, the seal lift bar 520. The lift jerk reduction spring 530 and the seal lift bar return spring 540 are compression springs and both are stud-mounted on a stud 680 fixed to and projecting upward from the lid frame 90. The stud 680 passes through a front-to-rear extending through slot 682 in the intermediate portion 644 of the seal lift bar 520 to enable front-to-rear movement of the seal lift bar 520 relative to the stud 680 during pivoting movement of the seal lift bar 520 about the free-rotating pin 660. The upper end of the lift jerk reduction spring 530 abuts a hex nut 684 threaded onto the top of the stud 680; as such, the lift jerk reduction spring 530 is sandwiched between the top 670 of the seal lift bar 520 and the bottom of the hex nut 684. The lower end of the seal lift bar return spring 540 rests on the lid frame 90; as such, the seal lift bar return spring 540 is sandwiched between the bottom 672 of the seal lift bar 520 and the top of the lid frame 90. As will be appreciated, the stud 680 enables each of the lift jerk reduction spring 530 and the seal lift bar return spring 540 to be compressed to a predetermined pre-load.

In operation, as the seal lift actuator 510 exerts an upward force on the front end 640 of the seal lift bar 520, the seal lift bar 520 rotates clockwise about the free-rotating pin 660, compressing the lift jerk reduction spring 530 and rotating the lid assembly 20 open about the hinge axis 210. Compression of the lift jerk reduction spring 530 enables the seal lift bar 520 and the seal lift actuator 510 to begin moving prior to the lid assembly 20 in a constant volume sub-phase of the opening state, see Fig. 5, as will be described in greater detail below. This has been found to effectively reduce sticking and/or jerking of the seal lift actuator 510 as the seal lift actuator 510 overcomes static friction for example in its wiper/piston seals, resulting in smooth movement of the lid assembly 20. When the seal lift actuator 510 exerts a downward force on the front end 640 of the seal lift bar 520 in its horizontal home position, the seal lift bar 520 rotates counterclockwise about the free-rotating pin 660, thereby causing the intermediate portion 644 of the seal lift bar 520 to compress the seal lift bar return spring 540 and the rear end 642 of the seal lift bar 520 to press up against the underside 652 of the stop member 650. This generates a net downward force on the rear hinge bracket 220, thereby moving the lid assembly 20 downwards. Referring to Fig. 24, the seal lift bar return spring 540 is pre-loaded such that it returns the seal lift bar 520 to its horizontal home position after the downward force from the seal lift actuator 510 on the front end 640 is released and the lid assembly 20 returns to the closed state.

The free-rotating pin 660 also pivotably couples the seal lift bar 520 to a front end 690 of the hinge bar 550. The hinge bar 550 extends rearwardly from the free-rotating pin 660 and then bends upward in an L-shape configuration. A rear end 692 of the hinge bar 550, in turn, is pivotably mounted via a free-rotating pin 694 to the rear frame upright 604. The hinge bar 550, which may be made of a polymeric material, is spring biased to a horizontal home position by the hinge bar return spring 560 beneath, or at a bottom 700 of, the rearwardly extending portion of the hinge bar 550. As shown in Fig. 24, the hinge bar return spring 560 is a compression spring and is stud-mounted on a stud 710 fixed to and projecting upward from the frame 12. The stud 710 passes through a front-to-rear extending through slot 712 in the rearwardly extending portion of the hinge bar 550 to enable front-to-rear movement of the hinge bar 550 relative to the stud 710 during pivoting movement of the hinge bar 550 about the free-rotating pin 694. The top of the hinge bar 550 abuts a hex nut 714 threaded onto the top of the stud 710. The lower end of the hinge bar return spring 560 rests on the frame 12; as such, the hinge bar return spring 560 is sandwiched between the bottom 700 of the hinge bar 550 and the top of the frame 12. As will be appreciated, the stud 710 enables the hinge bar return spring 560 to be compressed to a predetermined pre-load.

In operation, as the seal lift actuator 510 exerts a downward force on the lid assembly 20 via the seal lift bar 520 and the free-rotating pin 660, the hinge bar 550 rotates counterclockwise about the free-rotating pin 694, compressing the hinge bar return spring 560. The hinge bar return spring 560 is pre-loaded such that the hinge bar return spring 560 returns the hinge bar 550 to its horizontal home position when the downward force on the lid assembly 20 is released and holds the hinge bar 550 in the horizontal home position, for example, against gravity.

With continued reference to Figs. 20-24, the illustrated seal lift actuator 510 also includes the lift force balance spring 570 and the lid deceleration spring 580. The lift force balance spring 570 is a compression spring mounted on-axis on a rod 730 of the seal lift actuator 510. The lid deceleration spring 580 also is a compression spring mounted on-axis on the rod 730 but has a larger diameter than that of the lift force balance spring 570 such that the lid deceleration spring 580 surrounds and is concentric with the lift force balance spring 570. In the illustrated embodiment, a seal lift pivot 740 made of a polymeric material and configured in the form of a pivot block is attached to a lower end 734 of the rod 730. An upper end 742 of the seal lift pivot 740 retains the lift force balance spring 570 and the lid deceleration spring 580 on-axis with the seal lift actuator 510, and an opposite lower end 744 of the seal lift pivot 740 is pivotably coupled to the front end 640 of the seal lift bar 520 via the afore described free-rotating pin 608. An actuator cap 760 made of a polymeric material is attached to a lower end 762 of a cylinder body 764 of the seal lift actuator 510. The actuator cap 760 provides a compression surface against which the lift force balance spring 570 and the lid deceleration spring 580 press during a portion of the opening of the lid assembly 20. The actuator cap 760 also prevents the lift force balance spring 570 from contacting and/or damaging an internal rod wiper seal at the lower end of the cylinder body 764.

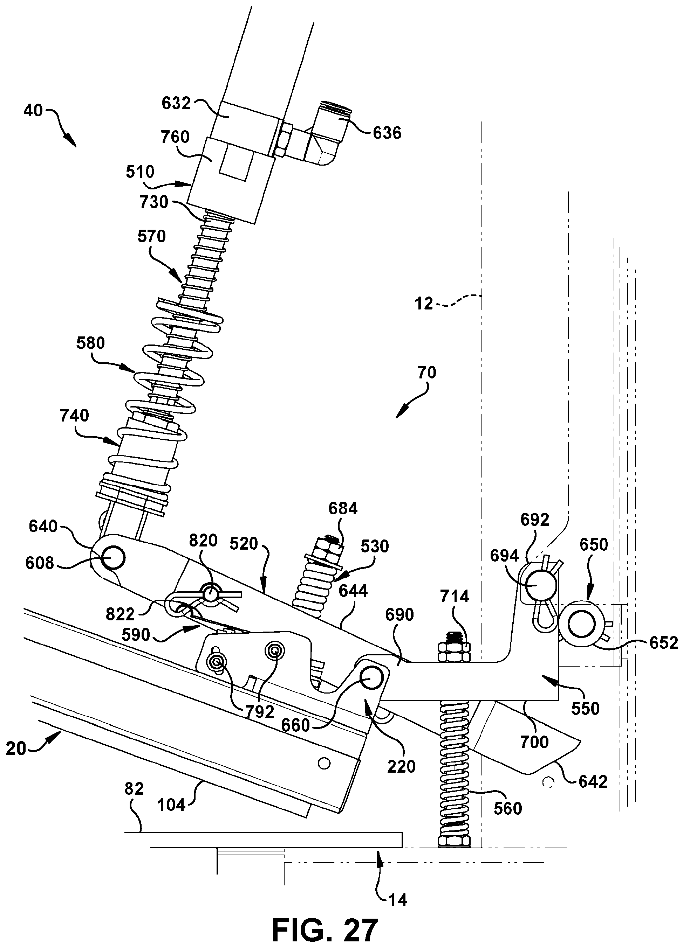

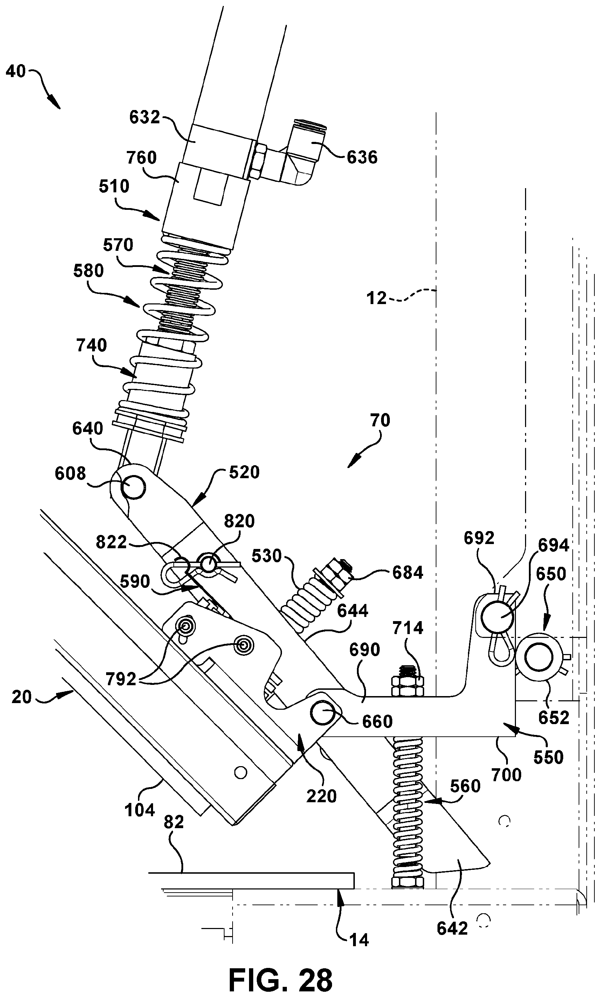

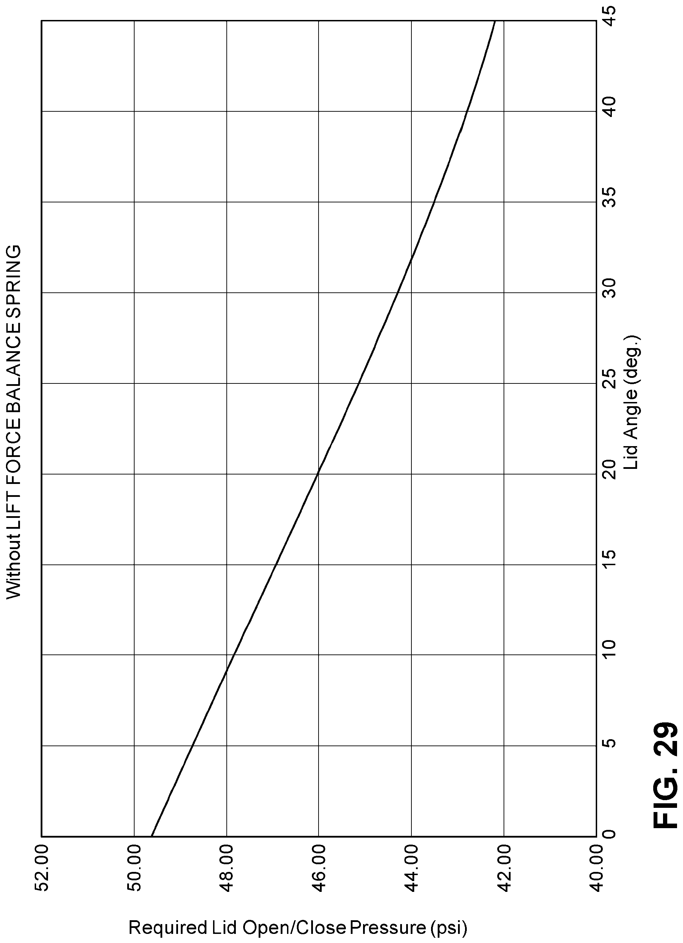

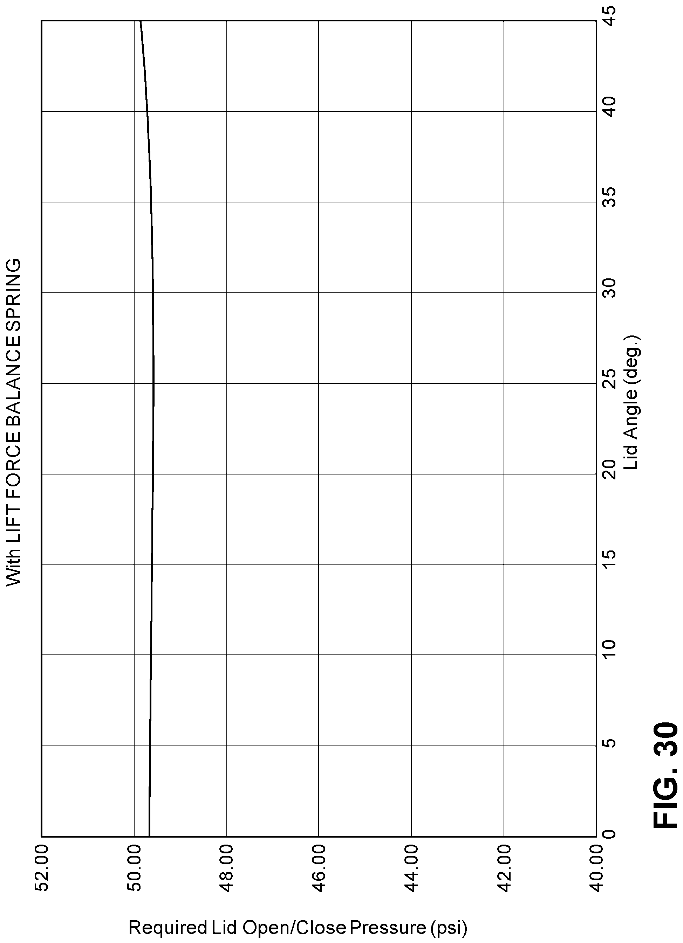

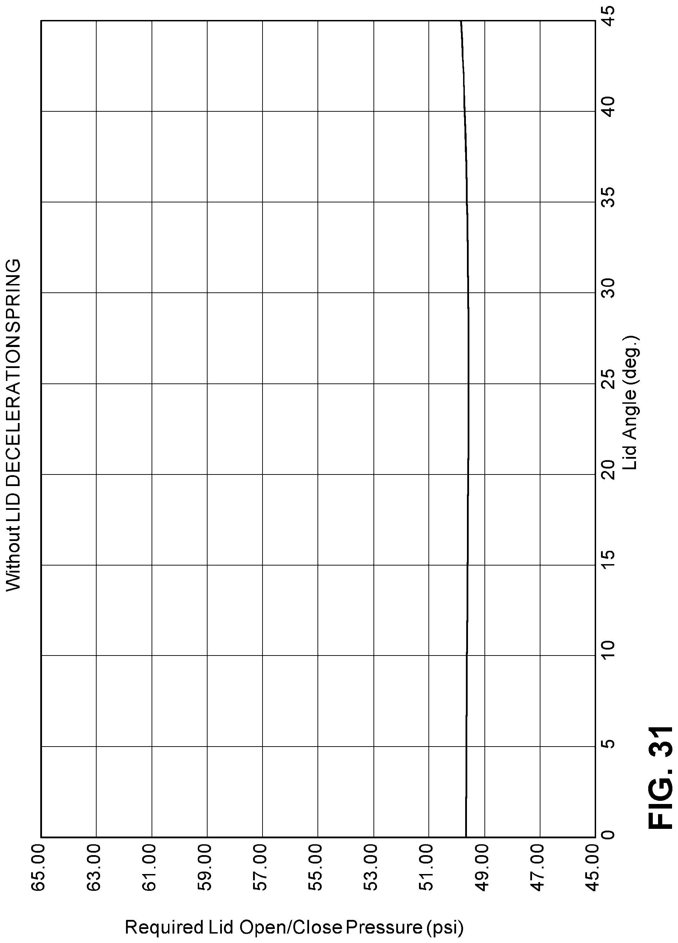

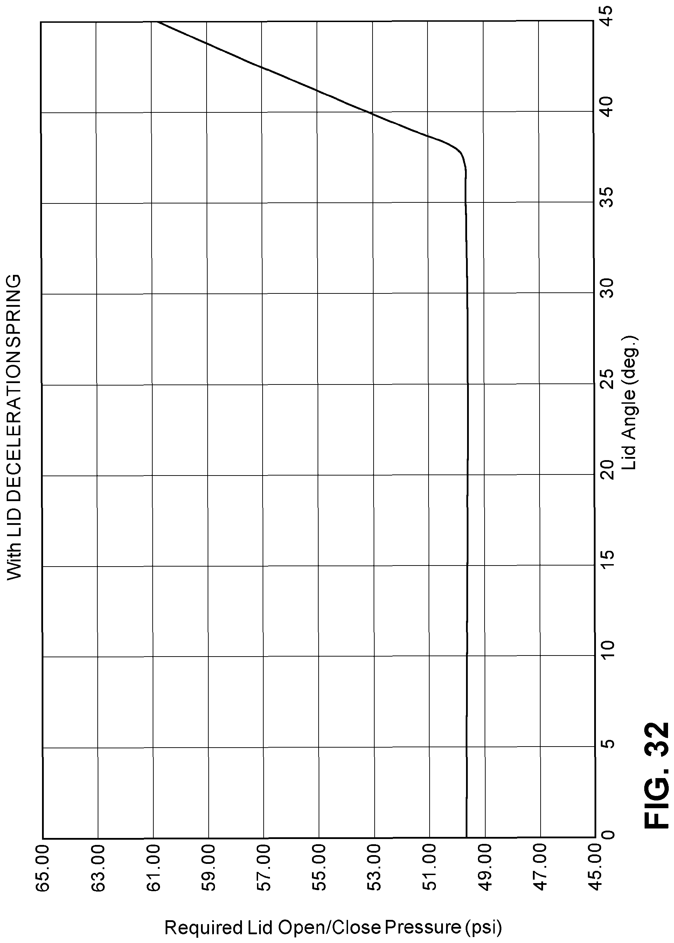

In operation, as the lid assembly 20 pivots about the free-rotating pin 660 in the opening and closing transitional states, the force vector angle between the seal lift actuator 510 and the lid assembly 20 changes, which in turn changes the required force vector magnitude to lift/lower the lid assembly 20. Because in the illustrated embodiment the lid opening/closing speed is controlled by pneumatic flowrate/pressure to the seal lift actuator 510, described in greater detail below, the lid speed may in some circumstances be erratic and/or inconsistent. The lift force balance spring 570 counteracts this change in the lift/lower force by holding the required pressure constant. The effect of the lift force balance spring 570 is illustrated by way of comparison of Fig. 29, which shows the seal lift actuator 510 without the lift force balance spring 570, and Fig. 30, which shows the seal lift actuator 510 with the lift force balance spring 570. Without the lift force balance spring 570, the required lid lift pressure drops, for example, from about 49.5 psi to about 42.5 psi, as the displacement angle of the lid assembly 20 increases. With the lift force balance spring 570, the required lid lift pressure remains substantially constant, for example, at about 49.5 psi, as the displacement angle of the lid assembly 20 changes. The lid deceleration spring 580 also aids in smooth movement of the lid assembly 20. The lid deceleration spring 580 is configured, when the lid assembly 20 has been angularly displaced a predetermined number of degrees before reaching the open position, to reduce the speed of the lid assembly 20. As the lid assembly 20 approaches its fully open orientation, see Figs. 27 and 28, the lid deceleration spring 580 begins to compress, increasing the required lid lift pressure. This decelerates the lid assembly 20 such that the lid assembly 20 comes to a gentle stop. The effect of the lid deceleration spring 580 is illustrated by way of comparison of Fig. 31, which shows the seal lift actuator 510 without the lid deceleration spring 580, and Fig. 32, which shows the seal lift actuator 510 with the lid deceleration spring 580. Without the lid deceleration spring 580, the required lid lift pressure remains constant, for example, at about 49.5 psi, as the lid assembly 20 approaches its fully open orientation. With the lid deceleration spring 580, the required lid lift pressure increases, for example from about 49.5 psi to about 61.0 psi, as the displacement angle of the lid assembly 20 approaches its fully open orientation. In the Fig. 32 example, the lid deceleration spring 580 reduces the speed of the lid assembly 20 when the lid assembly 20 has been angularly displaced about 37.5 degrees, that is about 7.5 degrees before reaching the open position of 45 degrees.

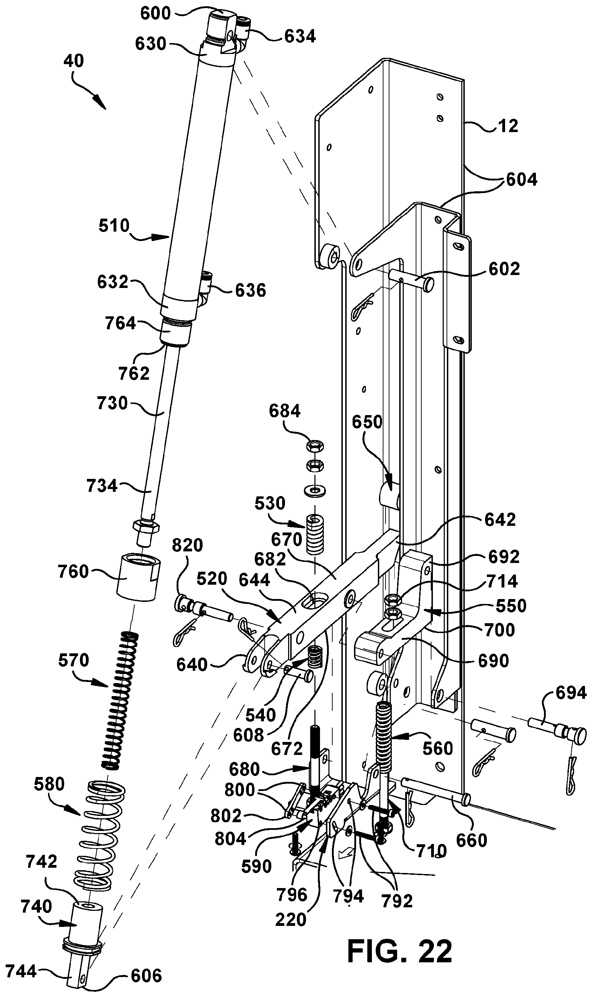

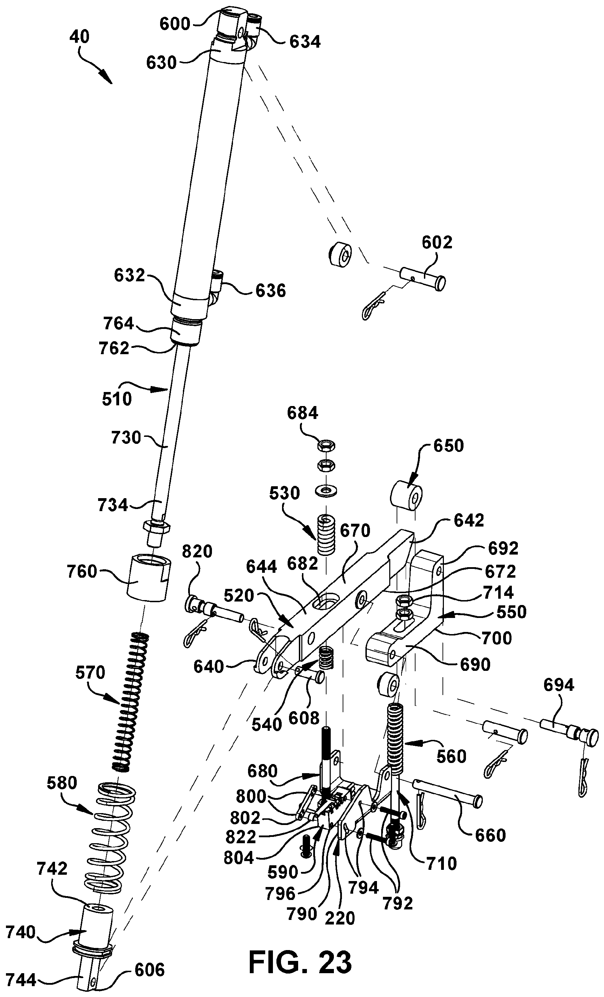

The illustrated rear actuation assembly 40 also includes the rear lid sealed detection switch 590, which is configured to detect displacement of the rear of the lid assembly 20 between the closed position shown in Fig. 24 and the sealed position shown in Fig. 25. The rear lid sealed detection switch 590 is mounted to a left side surface 790 of one of the upright members of the U-shape rear hinge bracket 220 by fasteners 792 that pass through through-openings 794 in the rear hinge bracket 220 and through-openings 796 (only one in view in Figs. 22 and 23) in the rear lid sealed detection switch 590, and thread into respective threaded openings 800 in a clamp bar 802 on a left side surface 804 of the rear lid sealed detection switch 590. The seal lift bar 520 includes a protruding member 820, in the illustrated embodiment in the form of a protruding pin, that is vertically above and in abutting contact with a distal end 822 of the rear lid sealed detection switch 590. As such, when the lift seal actuator 510 is actuated to urge the lift seal bar 520 counterclockwise, the protruding member 820 of the lift seal bar 520 presses down on the rear lid sealed detection switch 590 As will be described in greater detail below, the rear lid sealed detection switch 590 is configured such that, when depressed by the protruding member 820 of the seal lift bar 520, the rear lid sealed detection switch 590 provides an input to the controller 52 indicating that the lid assembly 20 is in a sealed state. One of the through-openings 794 in the rear hinge bracket 220 is in the form of an arc-shape slot as shown, enabling the mounting angle of the rear lid sealed detection switch 590 to be adjusted on the rear hinge bracket 220 such that the rear lid sealed detection switch 590 activates at a specific displacement of the seal lift bar 520. In the illustrated embodiment, the rear lid sealed detection switch 590 includes a limit switch that is configured to detect when the lid assembly 20 has reached a predetermined limit position corresponding to a predetermined sealing force applied to the elastomeric seal 104.

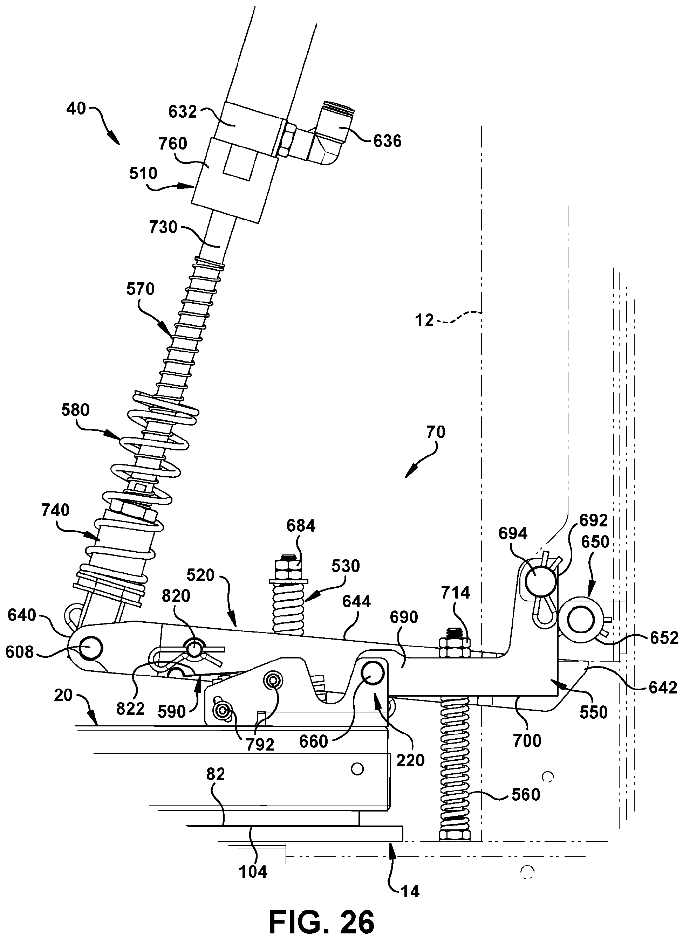

Turning again to the Fig. 5 state diagram, the rear actuation assemblies 40, 42 are configured to operate in a closed state as shown in Fig. 24, a sealed state as shown in Fig. 25, an opening/closing state as shown in Figs. 26 and 27, and an open state as shown in Fig. 28. Referring initially to Figs. 14 and 15, the closed state of the rear actuation assemblies 40, 42 coincides with the lid assembly 20 also being closed. In the illustrated embodiment, in the closed state the lid assembly 20, the seal lift bar 520, and the hinge bar 550 are all in a horizontal orientation, or home position. The elastomeric seal 104 is uncompressed and contacting the rim 82 of the processing basin 14. The rear end 642 of the seal lift bar 520 is in abutting contact with the underside 652 of the stop member 650 of the rear frame upright 604.

The sealed state of the rear actuation assemblies 40, 42 coincides with the lid assembly 20 also being sealed. Referring to Fig. 25, when the piston side or upper chamber 630 of the seal lift actuator 510 is pressurized, the seal lift actuator 510 exerts a downward force on the front end 640 of the seal lift bar 520. This rotates the seal lift bar 520 counterclockwise about the free-rotating pin 608, urging the front end 640 of the seal lift bar 520 downwards such that the seal lift bar 520 compresses the seal lift bar return spring 540. Meanwhile, the rear end 642 of the seal lift bar 520 pushes up against the underside 652 of the stop member 650, which generates the afore described net downward force on the hinge bracket 220, urging the lid assembly 20 downwards to compress the elastomeric seal 104 against the rim 82 of the processing basin 14. In this way, the rear actuation assemblies 40, 42 vertically displace the lid assembly 20 from the closed state to the sealed state. Also, as the seal lift bar 520 moves counterclockwise, the protruding member 820 of the seal lift bar 520 depresses the rear lid sealed detection switch 590, indicating to the controller 52 that the lid assembly 20 at the corresponding rear actuation assembly 40, 42 is sealed. Downward movement of the lid assembly 20 also causes the hinge bar 550 to rotate counterclockwise about the free-rotating pin 694 such that the hinge bar 550 compresses the hinge bar return spring 560. To unseal the lid assembly 20, the upper chamber 630 of the seal lift actuator 510 is exhausted. This causes the seal lift bar return spring 540 to rotate the seal lift bar 520 clockwise about the free-rotating pin 608, and the hinge bar return spring 560 to rotate the hinge bar 550 clockwise about the free-rotating pin 694, thereby returning the lid assembly 20, the seal lift bar 520, and the hinge bar 550 to their horizontal home positions and reverting the lid assembly 20 to the closed state shown in Fig. 24.

The next state of the lid assembly is the opening/closing state shown in Figs. 26 and 27. The opening/closing state has three sub-phases, see Fig. 5, consisting of constant volume sub-phase, a constant pressure sub-phase, and a deceleration sub-phase, which coincide with the compression states of the three active springs, that is, the lift jerk reduction spring 530, the lift force balance spring 570, and the lid deceleration spring 580, respectively.

Referring to Fig. 26, at the start of the opening phase, when the lower chamber 632 of the seal lift actuator 510 is pressurized, the seal lift actuator 510 exerts an upward force on the front end 640 of the seal lift bar 520. In this constant volume sub-phase, pressure builds in the lower chamber 632 of the seal lift actuator 510 at a substantially constant volume, rotating the seal lift bar 520 clockwise about the free-rotating pin 608 and compressing the lift jerk reduction spring 530 on top of the seal lift bar 520 located at the intermediate portion 644 of the seal lift bar 520. Meanwhile, the rear end 642 of the seal lift bar 520 moves downward and separates from the underside 652 of the stop member 650. In addition, the hinge bar return spring 560 holds the hinge bar 550 in a horizontal orientation as the seal lift bar 520 pivots about the free-rotating pin 608.

In the constant pressure sub-phase shown in Fig. 27, when the lower chamber 632 of the seal lift actuator 510 has built enough pressure for the upward force of the seal lift actuator 510 to overcome the weight of the lid assembly 20, the lid assembly 20 begins rotating about the hinge axis 210. As the seal lift pivot 740 at the lower end 734 of the rod 730 of the seal lift actuator 510 compresses the lift force balance spring 570 against the actuator cap 760 at the lower end 762 of the cylinder body 764 of the seal lift actuator 510, the lift force balance spring 570 keeps the lift force magnitude substantially constant even as the lift force angle changes, thereby holding the lid lift pressure substantially constant. The hinge bar return spring 560 continues to hold the hinge bar 550 in a horizontal orientation as the seal lift actuator 510 lifts the lid assembly 20.

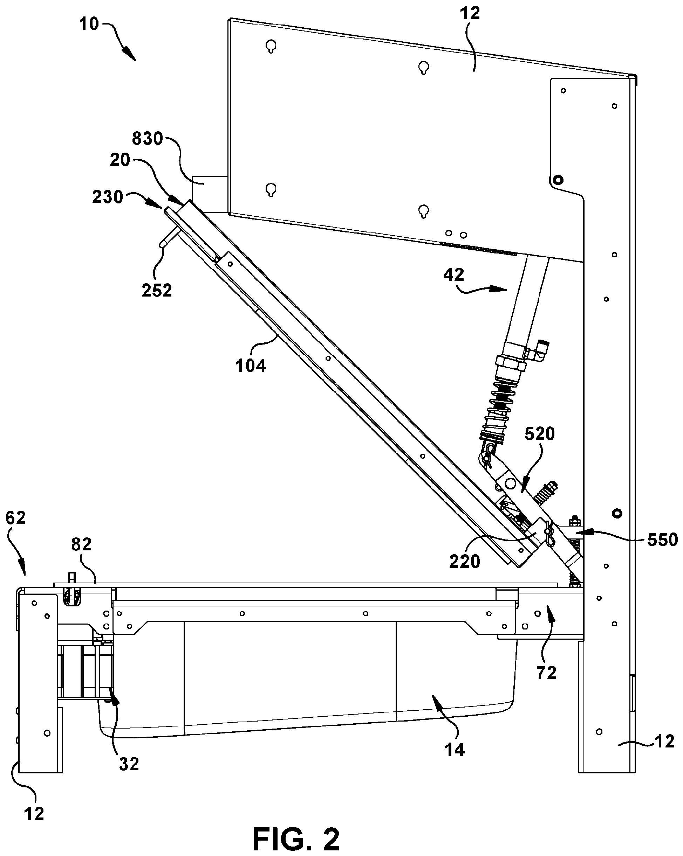

In the deceleration sub-phase, the lid assembly 20 is almost fully open. In this sub-phase, the seal lift pivot 740 compresses the lid deceleration spring 580 against the actuator cap 760 for the remainder of the opening phase, increasing the required lift force and pressure, see Fig. 32. The lid deceleration spring 580 decelerates the lid assembly 20 to a gentle stop. As shown in Fig. 2, the lid assembly 20 stops moving when it hits a rubber bumper 830 on the housing or frame 12 of the medical device processor 10, at which point the rear actuation assembly 40 and the lid assembly 20 are in the open state, see Fig. 5.

The closing phase of the rear actuation assemblies 40, 42 is the reverse of the opening phase and begins when the lid lift pressure in the lower chamber 632 of the seal lift actuator 510 is exhausted. In the illustrated embodiment, the lid assembly 20 is configured to automatically close by gravity, for example, without pressurization of the upper chamber 630 of the seal lift actuator 510. As will be appreciated, and referring again to Fig. 5, this automatic closing phase has three sub-phases consisting of a lid acceleration sub-phase, a constant pressure sub-phase, and a constant volume sub-phase, which coincide with decompression states of the three active springs, that is, the lid deceleration spring 580, the lift force balance spring 570, and the lift jerk reduction spring 530, respectively. When the closing phase is complete, the rear actuation assembly 40 and the lid assembly 20 are returned to a closed state, see Fig. 5.

Several advantages are realized by the medical device processor 10 including the front actuation assemblies 30, 32 and the rear actuation assemblies 40, 42. Referring again to Figs. 1-4, 8 and 20, the lid assembly 20 is configured for angular displacement about the hinge axis 210 between the closed position shown in Fig. 4 and the open position shown in Figs. 1-3. In the closed position, the lid assembly 20 covers the opening 80 of the processing basin 14. In the open position, the lid assembly 20 is at a non-zero angle relative to the lid assembly 20 in the closed position and the lid assembly 20 exposes the opening 80. As described above, the front actuation assemblies 30, 32 and the rear actuation assemblies 40, 42 are configured to displace the lid assembly 20 from the closed position to the sealed position shown in Fig. 12. In the sealed position, the lid assembly 20 compresses the elastomeric seal 104 between the lid assembly 20 and the rim 82 of the processing basin 14 to create a seal between the lid assembly 20 and the rim 82 to seal the opening 80 of the processing basin 14 from the exterior of the medical device processor 10. Thus, the front actuation assemblies 30, 32 and the rear actuation assemblies 40, 42 of the medical device processor 10 provide an advantage over prior systems in that they automate the sealing operation of the lid assembly 20. This automated sealing operation eliminates user error and thereby reduces the risk of leaks or process inefficacy.

Also, as described above, the front actuation assemblies 30, 32 in addition to providing a sealing function are also configured to latch a latch bolt 250 of the lid assembly 20 to lock the lid assembly 20 in the closed position and to release the latch bolt 250 to enable the lid assembly 20 to be displaced from the closed position to the open position. Further, the rear actuation assemblies 40, 42 in addition to providing a sealing function are also configured to angularly displace the lid assembly 20 about the hinge axis 210 of the lid assembly 20 from the closed position to the open position. Thus, the medical device processor 10 provides an advantage over prior systems in that, by having the front actuation assemblies 30, 32 and the rear actuation assemblies 40, 42 perform the sealing operation in the same mechanisms that latch/lock and open the lid assembly 20, respectively, system complexity is reduced.

Moreover, the front actuation assemblies 30, 32 and the rear actuation assemblies 40, 42 provide a modular system design which can be applied to alternate machine configurations. The front actuation assemblies 30, 32 and the rear actuation assemblies 40, 42 both combine a closing functionality and a sealing functionality. Thus, for example, the front actuation assemblies 30, 32 and the rear actuation assemblies 40, 42, and optionally also the lid assembly 20, can easily be installed on a prior existing medical device processor having a different size basin and/or different elastomeric seal configuration since the sealing aspect does not involve a separate component altogether but rather is incorporated into the front actuation assemblies 30, 32 and the rear actuation assemblies 40, 42 themselves. The front actuation assemblies 30, 32 and the rear actuation assemblies 40, 42 can be installed on a wider or narrower medical device processor, for example, and the front actuation assemblies 30, 32 and the rear actuation assemblies 40, 42 will still function in the same manner.

In some embodiments, actuation assemblies comprising a seal actuator 310 and an unlatch actuator 320 could replace the rear actuation assemblies 40, 42 at the rear-left corner 70 and the rear-right corner 72. For example, where the lid assembly 20 has an alternative hinge arrangement and four latch bolts 250 at its four corners, actuation assemblies comprising a seal actuator 310 and an unlatch actuator 320 could be arranged at the four corners of a processing basin so that the four actuation assemblies would pull down on the four latch bolts 250 at the four corners of the lid assembly 20, thereby providing four-point sealing and four-point locking of the lid assembly 20 to the processing basin. It is also contemplated that for passthrough configuration type medical device processors, the seal actuator 310 and the unlatch actuator 320 may be implemented at four different corners. In this way, the four actuation assemblies, each having a seal actuator 310 and an unlatch actuator 320, would provide four-point sealing and four-point locking of a lid assembly of a passthrough configuration type medical device processor.

Another advantage of the rear actuation assemblies 40, 42 is the mounting of the lift force balance spring 570 and the lid deceleration spring 580 on-axis on the rod 730 of the rear actuation assemblies 40, 42. This further adds to the modularity aspect of the lid assembly 20, enabling the lid assembly 20 to be installed on prior medical device processors.

The medical device processor 10 according to the invention also includes the front lid sealed detection switches 340 that are configured to detect displacement of the front of the lid assembly 20 between the closed position and the sealed position, and the rear lid sealed detection switches 590 that are configured to detect displacement of the rear of the lid assembly 20 between the closed position and the sealed position. Thus, the medical device processor 10 provides an advantage over prior systems in that the front lid sealed detection switches 340 and the rear lid sealed detection switches 590 directly monitor displacement of the lid assembly 20 during the sealing operation, in the illustrated embodiment at all four corners thereof, that is, the front-left corner 60, the front-right corner 62, the rear-left corner 70 and the rear-right corner 72. This enables the medical device processor 10 to directly monitor for the most common failure modes, that is, pneumatic, mechanism, or obstructions, that may prevent the full sealing force from being applied to the elastomeric seal 104. The lid sealed detection switch monitoring therefore ensures that the lid assembly 20 is compressing the elastomeric seal 104 against the rim 82 of the processing basin 14 adequately at the four corners.

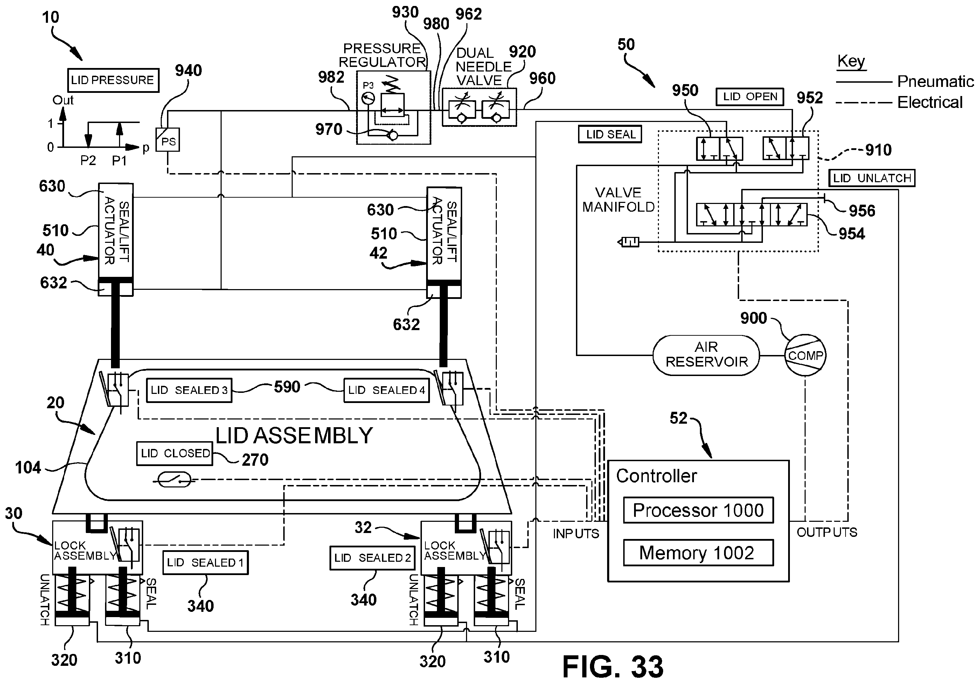

Turning now to Figs. 33-37, the pneumatic system 50 and the controller 52 according to an embodiment of the invention will now be described. As was noted above, the illustrative seal actuator 310, unlatch actuator 320 and seal lift actuator 510 are pneumatic type actuators but alternatively could comprise a hydraulic or electrical type actuator. It will be appreciated, then, that the pneumatic system 50 may be replaced with a hydraulic system or an electrical system, and the controller 52 may be configured accordingly to control the hydraulic components and/or the electrical components of such systems. As shown in Fig. 33, the pneumatic system 50 includes a compressor 900, a valve manifold 910, a bidirectional flow control valve 920, a pressure regulator 930, and a lid pressure switch 940, each of which is described in turn below, followed by a description of the controller 52 and an exemplary operation of the medical device processor 10.

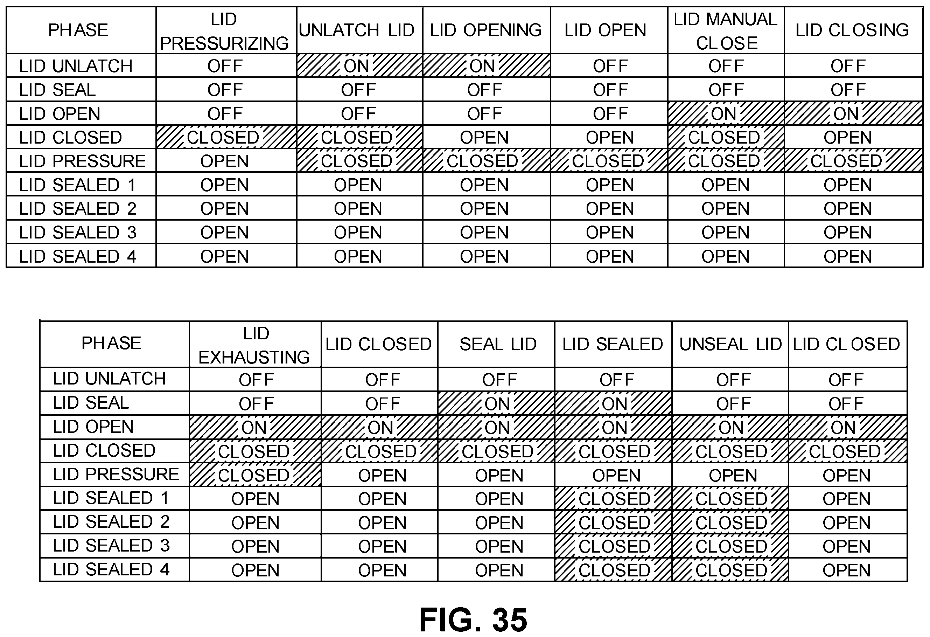

The illustrated valve manifold 910 is a four-valve pneumatic manifold that receives pressurized air from the compressor 900 and is made up of a lid seal valve 950, a lid open valve 952, a lid unlatch valve 954, and a plugged valve 956. The lid seal valve 950 is a 3/2 normally closed (NC) valve in fluid communication with the seal actuators 310 of the front actuation assemblies 30, 32 and the upper chamber 630 of the seal lift actuators 510 of the rear actuation assemblies 40, 42. As shown in Fig. 35, the lid seal valve 950 is ON during the sealed state of the lid assembly 20. The lid open valve 952 is a 3/2 normally open (NO) valve in fluid communication with the bidirectional flow control valve 920. As shown in Fig. 35, the lid open valve 952 is ON during the closing, closed, and sealed states of the lid assembly 20. The lid unlatch valve 954 is a 5/3 valve (mid-position exhausting), control port 1, in fluid communication with the unlatch actuators 320 of the front actuation assemblies 30, 32. As shown in Fig. 35, the lid unlatch valve 954 is ON during the opening state of the lid assembly 20, which corresponds to the unlatch state of the front actuation assemblies 30, 32. The plugged valve 956 is a 5/3 valve (mid-position exhausting), control port 2, that is reserved for additional unlatch actuators 320, for example, on a medical device processor having a passthrough configuration.

The bidirectional flow control valve 920 is an adjustable dual needle valve having an inlet 960 in fluid communication with the lid open valve 952, and an outlet 962 in fluid communication with the pressure regulator 930. The bidirectional flow control valve 920 provides two adjustments, one for controlling supply flowrate into the lower chambers 632 of the seal lift actuators 510 during opening of the lid assembly 20, and one for controlling exhaust flowrate from the lower chambers 632 during closing of the lid assembly 20. Thus, the bidirectional flow control valve 920 enables independent control of supply and exhaust flowrates to and from the lower chambers 632 of the seal lift actuators 510, thereby enabling control of the respective lid opening and lid closing speeds.

The pressure regulator 930 is an adjustable pressure regulator with an over-pressurization valve 970. The pressure regulator 930 has an inlet 980 in fluid communication with the bidirectional flow control valve 920 and an outlet 982 in fluid communication with the lower chambers 632 of the seal lift actuators 510. The pressure regulator 930 is configured to regulate the source pressure from the compressor 900 to a set predetermined pressure P3, where P3 is a sufficient pressure for the rear actuation assemblies 40, 42 to angularly displace the lid assembly 20 from the closed position to the open position and to hold the lid assembly 20 in the open position against gravity and the spring forces from the lift force balance spring 570 and the lid deceleration spring 580. As will be described in greater detail below, this limits the amount of lift force to only what is required to hold the lid assembly 20 open, greatly reducing force magnitudes at any pinch points. Also, in the case where the pressure at the outlet 982 exceeds the predetermined pressure P3, the over-pressurization valve 970 of the pressure regulator 930 will vent air at the outlet 982 until the pressure equals the predetermined pressure P3. This enables the lid assembly 20 to be closed manually, as the manual closing force would compress the air in the lower chambers 632 of the seal lift actuators 510, thereby causing the pressure to exceed the predetermined pressure P3.

The lid pressure switch 940 is an adjustable normally open (NO) pressure switch with a configurable setpoint P1 and reset point P2. The lid pressure switch 940 is in fluid communication with, and in teed off connection between, the pressure regulator 930 and the lower chambers 632 of the seal lift actuators 510, thereby enabling the lid pressure switch 940 to directly measure lid lift pressure. The setpoint P1 is a predetermined pressure P1 that is less than what is required to overcome the weight of the lid assembly 20. Thus, and as will be described in greater detail below, if the lid assembly 20 was closed and the front actuation assemblies 30, 32 unlatched, and then pressure in the lower chambers 632 of the seal lift actuators 510 was increased from zero to the predetermined pressure P1, then the lift jerk reduction spring 530 would begin to compress but the lid assembly 20 would remain horizontally oriented (closed). The setpoint P1 is used by the controller 52 during the opening phase to initiate the unlatch state of the front actuation assemblies 30, 32. The reset point P2 is a predetermined pressure P2 that is less than the predetermined pressure P1 of setpoint P1, and is a lower pressure than what is required to latch the front actuation assemblies 30, 32 during the closing phase. Thus, and as will be described in greater detail below, if the lid assembly 20 were open and the lid lift pressure in the lower chambers 632 of the seal lift actuators 510 was reduced from the predetermined pressure P3 to the predetermined pressure P2, then the lid assembly 20 would close and latch. The reset point P2 is used by the controller 52 during the closing phase to detect obstructions.

The controller 52 is configured to control aspects of the closing, sealing, and opening operations of the lid assembly 20. In the Fig. 33 example, the controller 52 is configured to receive inputs from the lid closed switch 270, the front lid sealed detection switches 340, the rear lid sealed detection switches 590, and the lid pressure switch 940, and to generate outputs to control the compressor 900 and the valve manifold 910 to supply and/or exhaust pressurized air at an appropriate pneumatic flowrate/pressure to and/or from the seal actuators 310, the unlatch actuators 320 and the seal lift actuators 510. The controller 52 may include a processor 1000 for receiving the inputs and generating outputs based on the inputs. The processor 1000 may include any suitable microprocessor, control processing unit (CPU), control circuitry, or the like. A memory 1002 may also be provided as part of the controller 52. The memory 1002 may contain stored data pertaining to operation of the medical device processor 10 that is used by the processor 1000 in generating the outputs of the controller 52. For example, the memory 1002 may be configured to store data or a look-up table pertaining to required control outputs for yielding the appropriate pressures at which the seal actuators 310, the unlatch actuators 320 and the seal lift actuators 510 are to operate.

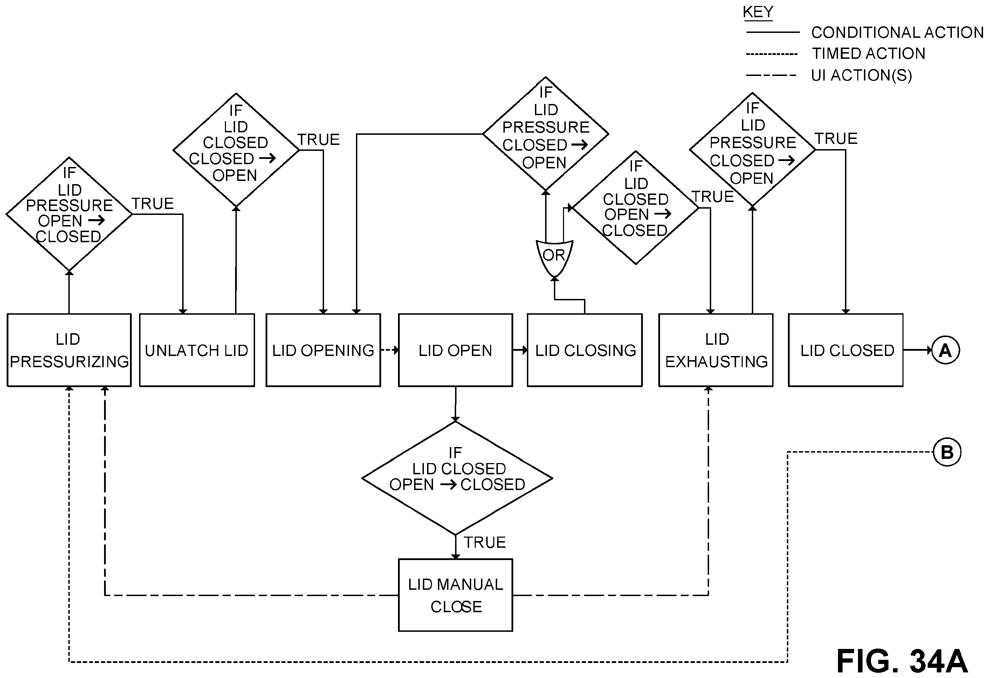

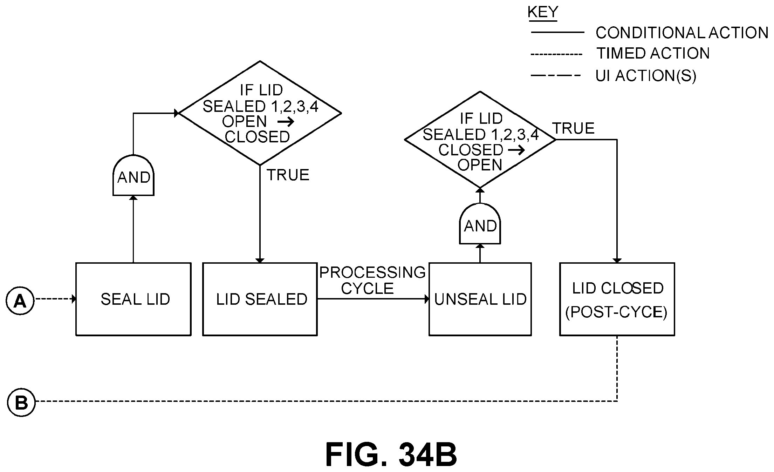

Figs. 34A and 34B show a state diagram of how the controller 52 interfaces with the components of the medical device processor 10 in accordance with an embodiment of the invention. Fig. 35 shows a chart identifying the state of a component identified in the left column during a phase of the medical device processor 10 identified in the top row in accordance with an embodiment of the invention. The components listed in the left column of Fig. 35 correspond to the components shown in the Fig. 33 schematic diagram and the Figs. 34A and 34B state diagram.

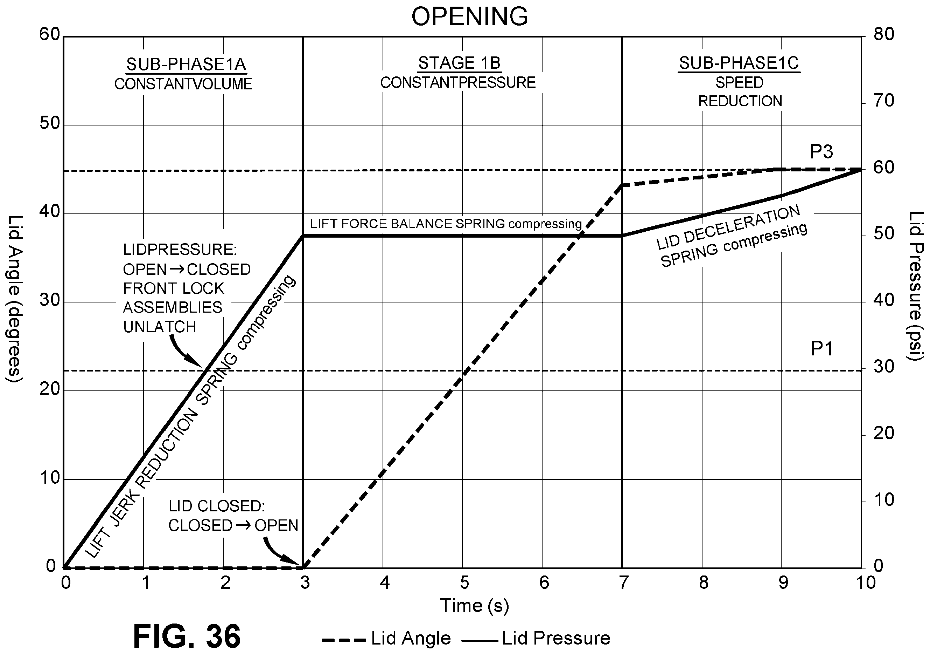

Fig. 36 is a graph showing the lid angle and the lid lift pressure of the lid assembly 20 versus time during an opening phase of the medical device processor 10 in accordance with an embodiment of the invention. Prior to opening, when the lid assembly 20 is closed, the lid closed switch 270 is closed and the lid open valve 952 is ON (the lid open valve 952 is normally open, NO). As such, the lid open valve 952 has allowed the lower chambers 632 of the seal lift actuators 510 to exhaust the lid lift pressure. To begin the opening phase, see Fig. 36, the controller 52 transmits an output to the lid open valve 952 to turn OFF. As such, the lid open valve 952 allows pressure to enter the lower chambers 632 of the seal lift actuators 510, and the seal lift actuators 510 thereby begin compressing the lift jerk reduction springs 530. Meanwhile, the controller 52 monitors the lid pressure switch 940 until the lid pressure switch 940 closes, thus providing an input to the controller 52 that the lid lift pressure in the lower chambers 632 has reached the predetermined pressure P1 (setpoint P1), which in the Fig. 36 example is about 30 psi. Based on such input, the controller 52 transmits an output to the lid unlatch valve 954 to turn ON (the lid unlatch valve 954 is normally closed, NC). As such, the lid unlatch valve 954 allows pressure to the unlatch actuators 320, thereby causing the unlatch actuators 320 to release the latch bolts 250 of the lid assembly 20. The seal lift actuators 510 continue to compress the lift jerk reduction springs 530 and the controller 52 continues to monitor the lid closed switch 270. When the lid assembly 20 starts to open, which is at about 3 seconds and at a lid lift pressure of about 50 psi in the Fig. 36 example, the lid closed switch 270 opens, thereby providing an input to the controller 52 that the lid assembly 20 has started to open. The seal lift actuators 510 gradually open the lid assembly 20, compressing the lift force balance springs 570 and the lid deceleration springs 580 in the process. After a predetermined time delay, the controller 52 transmits an output to the lid unlatch valve 954 to turn OFF and the lid assembly 20 is considered OPEN.

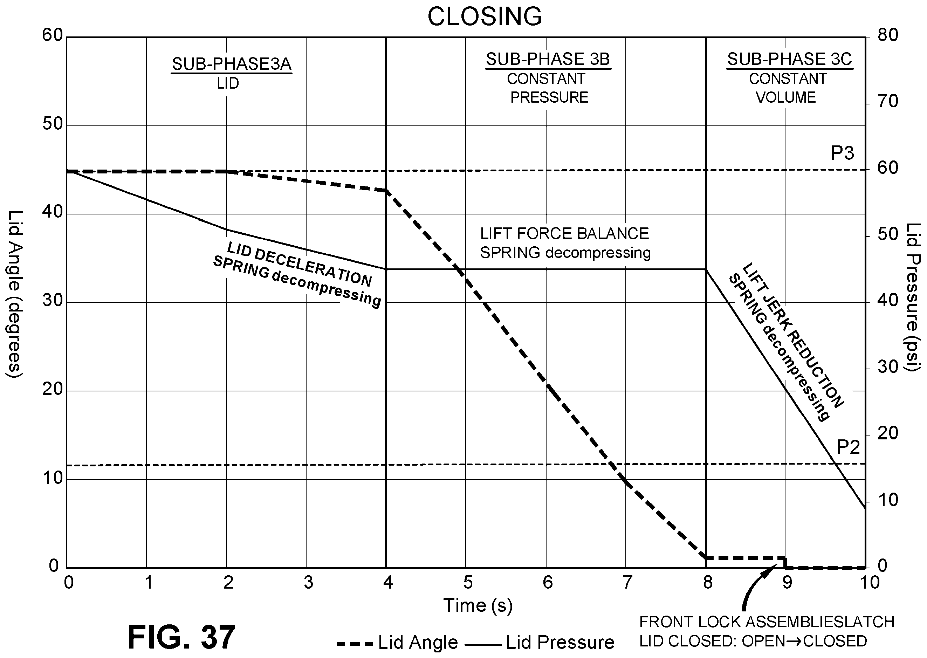

Fig. 37 is a graph showing the lid angle and lid lift pressure of the lid assembly 20 versus time during a closing phase of the medical device processor 10 in accordance with an embodiment of the invention. While the lid assembly 20 is open, a user interface (not shown) of the medical device processor 10 provides the option to either close the lid assembly 20 automatically or manually. If the user chooses to close the lid assembly 20 automatically, the controller 52 will begin the closing phase by transmitting an output to the lid open valve 952 to turn ON. As such, the lid open valve 952 begins allowing the lower chambers 632 of the seal lift actuators 510 to exhaust the lid lift pressure. In the Fig. 37 example, about 2 seconds after the lid deceleration springs 580 started decompressing, the lid angle of the lid assembly 20 begins to decrease, that is, from the open position of 45 degrees downward. As the lid assembly 20 continues toward the closed position and the lid angle decreases, the lid deceleration springs 580 decompress, the lift force balance springs 570 decompress, and the lift jerk reduction springs 530 decompress. Meanwhile, the controller 52 monitors the lid closed switch 270 and the lid pressure switch 940 for a state change. At about 9 seconds in the Fig. 37 example, as the lift jerk reduction springs 530 continue to decompress and the latch assemblies 330 of the front actuation assemblies 30, 32 latch with the latch bolts 250 of the lid assembly 20, the lid closed switch 270 changes from open to closed (before the lid pressure switch 940 changes from closed to open), indicating that the lid assembly 20 has been successfully latched without any impedances. The lid closed switch 270, having changed from open to close, provides an input to the controller 52 that the lid assembly 20 is closed. The controller 52 continues to monitor the lid pressure switch 940 until the lid pressure switch 940 opens, thus providing an input to the controller 52 that the lid lift pressure in the lower chambers 632 has dropped below the predetermined pressure P2 (reset point P2), which in the Fig. 37 example is about 15 psi. The lid assembly 20 now is considered closed.

Conversely, if the lid pressure switch 940 changes from closed to open first (that is, before the lid closed switch 270 changes from open to closed), then this indicates that an obstruction is impeding closing and latching of the lid assembly 20, that is, the lid lift pressure in the lower chambers 632 of the seal lift actuators 510 dropped below the predetermined pressure P2 (reset point P2) before the latch assemblies 330 could latch with the latch bolts 250 and the lid closed switch 270 could indicate the lid assembly 20 is closed. The lid pressure switch 940, having changed from closed to open, provides an input to the controller 52 that closing of the lid assembly 20 has been impeded. In this case, the controller 52 transmits an output to the lid open valve 952 to turn OFF. As such, the lid open valve 952 allows pressure to enter the lower chambers 632 of the seal lift actuators 510, and the seal lift actuators 510 thereby re-open the lid assembly 20. In some embodiments, the allowed pressure to the lower chambers 632 of the seal lift actuators 510 may be configured to merely stop further movement of the lid assembly 20.

Alternatively, the user can choose to close the lid assembly 20 manually. During manual closing of the lid assembly, should the lid lift pressure in the lower chambers 632 of the seal lift actuators 510 exceed the predetermined pressure P3, which in the Fig. 37 example is about 60 psi, the over-pressurization valve 970 of the pressure regulator 930 will vent at the outlet 982 until the pressure equals the predetermined pressure P3. When the controller 52 receives the input from the lid closed switch 270 that the lid closed switch 270 has changed from open to close, the controller 52 transmits an output to the lid open valve 952 to turn ON. As such, the lid open valve 952 allows the lower chambers 632 of the seal lift actuators 510 to exhaust the lid lift pressure.

The user interface then provides two options, either to re-open the lid assembly 20, or to seal the lid assembly 20 and start the processing cycle. If the user selects the option to re-open the lid assembly 20, the controller 52 moves to the opening state, see Fig. 36. If the user selects the option to seal the lid assembly 20, the controller 52 moves to the sealed state.

After the lid assembly 20 is closed, the controller 52 transmits an output to the lid seal valve 950 to turn ON. As such, the lid seal valve 950 allows pressure to the seal actuators 310 of the front actuation assemblies 30, 32, thereby causing the seal actuators 310 to rotate the latch assemblies 330, as above described, to pull down on the latch bolts 250, 252 of the lid assembly 20 and thereby seal the front of lid assembly 20. At the same time, the lid seal valve 950 allows pressure to the upper chambers 630 of the seal lift actuators 510 of the rear actuation assemblies 40, 42, thereby causing the seal lift actuators 510 to rotate the seal lift bars 520 as above described, to vertically displace the lid assembly 20 and thereby seal the rear of lid assembly 20. Meanwhile, the controller 52 monitors the front lid sealed detection switches 340 and the rear lid sealed detection switches 590 until they all are closed, indicating to the controller 52 that the lid assembly 20 is sealed. At this stage the lid assembly 20 is considered sealed, and the processing cycle begins. After the processing cycle is complete, the controller 52 transmits an output to the lid seal valve 950 to turn OFF. As such, the lid seal valve 950 allows the seal actuators 310 and the upper chambers 630 of the seal lift actuators 510 to exhaust, thereby causing the latch assembly return springs 332, seal lift bar return springs 540, and hinge bar return springs 560 to return the latch assemblies 330 and seal lift bars 520 to their horizontal home positions, causing the lid assembly 20 to revert to its closed state. Meanwhile, the controller 52 monitors the front lid sealed detection switches 340 and the rear lid sealed detection switches 590 until they all are open, thereby indicating to the controller 52 that the lid assembly 20 is unsealed and again in a closed state and the lid assembly 20 ready to be opened.

It will be appreciated that the pneumatic system 50 of the medical device processor 10 need not be limited to the above-described bidirectional flow control valve 920, pressure regulator 930, and pressure switch 940, and other embodiments are contemplated. For example, the bidirectional flow control valve 920 could be replaced with a fixed orifice, and adjustment of the opening/closing speed may be implemented by other features of the pneumatic system 50 or omitted. The pressure regulator 930 need not incorporate the over-pressurization valve 970. In some embodiments, the over-pressurization valve 970 can be implemented elsewhere in the pneumatic system 50, for example, in the pneumatic line(s) between the lid open valve 952 and the lower chambers 632 of the seal lift actuators 510. The pressure switch 940 could be replaced with a pressure transducer, requiring the controller 52 to process the analog data and compare to setpoints.

Several advantages are realized by the medical device processor 10 including the controller 52 configured to monitor inputs and generate outputs in accordance with the invention. Referring again to Figs. 1-4 and 33-37, the medical device processor 10 in accordance with the invention includes the rear actuation assemblies 40, 42 being configured to be pressurized with a lid lift pressure, for example by the pneumatic system 50, to angularly displace the lid assembly 20 about the hinge axis 210 of the lid assembly 20 from a closed position or state to an open position or state, wherein in the closed position the lid assembly 20 covers the opening 80, and wherein in the open position the lid assembly 20 is at a non-zero angle relative to the lid assembly 20 in the closed position and the lid assembly 20 exposes the opening 80. Further, the lid assembly 20 is configured to be angularly displaced about the hinge axis 210 from the open position to the closed position, for example by gravity. The controller 52 as above described is configured to control pressurization of the rear actuation assemblies 40, 42 and to monitor the lid lift pressure of the rear actuation assemblies 40, 42 as the lid assembly 20 moves from the open position to the closed position. Thus, as the lid assembly 20 moves from the open position toward the closed position, if the lid lift pressure of the rear actuation assemblies 40, 42, for example that is exhausted from the lower chambers 632 of the seal lift actuators 510, reaches or falls below the predetermined pressure P2 prior to the closing of the lid assembly 20, thereby indicating impedance of the closing of the lid assembly 20, then the controller 52 pressurizes the rear actuation assemblies 40, 42 with a sufficient pressure to stop further movement of the lid assembly 20 or to angularly displace the lid assembly 20 to the open position. Obstruction detection is performed by monitoring the lid lift pressure of the rear actuation assemblies 40, 42 rather than by, for example, monitoring the large open volume of the swing path of the lid assembly 20 with tape switches or light screens as in prior systems. The medical device processor 10 is thus cost advantageous over prior systems because the controller 52 need only monitor the lid lift pressure of the rear actuation assemblies 40, 42, for example by means of the pressure switch 940, which is also significantly simpler than tape switches or light screens and requires significantly less infrastructure.

The medical device processor 10 also provides an automated open and close method that utilizes the lift force balance spring 570 and the lid deceleration spring 580 of the rear actuation assemblies 40, 42 in conjunction with the supply and exhaust features of the pneumatic system 50. The lift force balance spring 570 maintains a constant lift force magnitude/pressure for the majority of the open/close operation, even as the lift force angle changes. The lid deceleration spring 580 increases lift force magnitude/pressure as the lid assembly 20 approaches the end of the open operation; and, conversely, decreases the pressure as the lid assembly 20 starts its close operation.

The bidirectional flow control valve 920 of the pneumatic system 50 independently controls supply and exhaust flowrate, controlling open and close speed of the lid assembly 20. The pressure regulator 930 is set at a predetermined pressure P3 that holds the lid assembly 20 in an open state against gravity and the lift forces from the lift force balance spring 570 and the lid deceleration spring 580, limiting the lid lift force to only what is required to hold the lid assembly 20 open and greatly reducing force magnitudes at any pinch points. The pressure regular 930 includes an over-pressurization valve 970 that exhausts the rear actuation assemblies 40, 42 when the lid lift pressure exceeds the predetermined pressure P3, thereby allowing the lid assembly 20 to also be closed manually.

The lid pressure switch 940 of the pneumatic system 50 has an adjustable setpoint P1 and reset point P2. The setpoint P1 is a predetermined pressure P1 that is less than what is required to overcome the weight of the lid assembly 20 during the opening phase. When reached, the lid pressure switch 940 provides an input to the controller 52 to unlatch the front actuation assemblies 930. The reset point P2 is a predetermined pressure P2 that is less than what is required to latch the front actuation assemblies 30, 32 during the closing phase. If lid lift pressure reaches or falls below the predetermined pressure P2, the lid pressure switch 940 provides an input to the controller 52 that closing of the lid assembly 20 is being obstructed.

The lift jerk reduction spring 530, the lift force balance spring 570 and the lid deceleration spring 580 provide a predictable and repeatable pressure profile of the lid assembly 20. The controller 52 can thus use the pressure profile as a monitored parameter; that is, by monitoring the pressure profile via the lid pressure switch 940 during a closing operation of the lid assembly 20, the controller 52 is able to detect an obstruction and/or that the user desires to manually close the lid assembly 20.

Although the invention has been shown and described with respect to a certain embodiment or embodiments, it is obvious that equivalent alterations and modifications will occur to others skilled in the art upon the reading and understanding of this specification and the annexed drawings.

## Claims

1. A medical device processor (10), comprising:
a basin (14) having an opening (80) for receiving therein a medical device for processing and a rim (82) at the perimeter of the opening (80);
a lid frame (90); and,
a seal window assembly (100) removably mounted to the lid frame (90);
wherein the seal window assembly (100) includes a window (102) and an elastomeric seal (104) fixed to the window (102);
wherein the lid frame (90) with the seal window assembly (100) mounted thereto is displaceable relative to the rim (82) of the basin (14) between an open position and a sealed position, wherein in the open position the seal window assembly (100) is at a non-zero angle relative to the seal window assembly (100) in the sealed position and the seal window assembly (100) exposes the opening (80), and wherein in the sealed position the elastomeric seal (104) of the seal window assembly (100) is in sealing contact with the rim (82) of the basin (14) to create a seal between the window (102) and the rim (82) to seal the opening (80) of the basin (14) from the exterior of the medical device processor (10); and
**characterised in that**
the lid frame (90) includes a rail structure (120) configured for slidably guiding an edge structure (130) of the window (102) of the seal window assembly (100) to removably mount the seal window assembly (100) to the lid frame (90).

2. The medical device processor (10) of claim 1, wherein the rail structure (120) is lined with cushioning strips (190) secured thereto against which a surface (202) of the edge structure (130) of the window (102) slides during sliding movement relative to the rail structure (120).

3. The medical device processor (10) of claim 2, wherein the cushioning strips (190) include one or more of felt strips and fiberglass.

4. The medical device processor (10) of any of claims 1-3, wherein the rail structure (120) includes at least one support rail and the edge structure (130) includes at least one edge.

5. The medical device processor (10) of any of claims 1-4, wherein the rail structure (120) includes left, right, and rear support rails (122, 124, 126), and the edge structure (130) includes left, right, and rear edges (132, 134, 136), and the lid frame (90) further includes an open front end (150) that is configured such that the left, right, and rear edges (132, 134, 136) are insertable and removable through the open front end (150) and slidably guided by the respective left, right, and rear support rails (122, 124, 126).

6. The medical device processor (10) of any of claims 1-5, wherein the rail structure (120) has a concave structure, and the edge structure (130) of the window (102) has a convex structure such that the edge structure (130) of the window (102) is slidably movable within the concave structure of the rail structure (120).

7. The medical device processor (10) of claim 6, wherein the concave structure is C shape in cross section and cushioning strips (190) are secured to an underside (192) of one of the arms of the C shape structure against which a surface (202) of the convex structure of the window (102) slides during sliding movement within the concave structure.

8. The medical device processor (10) of any of claims 1-7, wherein the lid frame (90) is mounted to the basin (14) for pivotable movement about a hinge axis (210) and the seal window assembly (100) is removably mounted to the lid frame (90) for slidable movement in a direction perpendicular to the hinge axis (210).

9. The medical device processor (10) of any of claims 1-8, wherein the lid frame (90) further includes an open front end (150) that is configured such that the edge structure (130) of the window (102) is insertable and removable through the open front end (150) and slidably guided by the rail structure (120).

10. The medical device processor (10) of any of claims 1-9, further comprising a cover panel (230) removably mounted to a front support member (160) of the lid frame (90) to support a front edge (240) of the edge structure (130) of the window (102) and prevent sliding movement of the window (102) relative to the rail structure (120).

11. The medical device processor (10) of any of claims 1-10, wherein the rim (82), the lid frame (90), and the elastomeric seal (104) are each annular in shape.

12. The medical device processor (10) of any of claims 1-11, wherein the seal window assembly (100) is configured such that when the lid frame (90) is in the sealed position the window (102) enables viewing of the interior of the basin (14).

## Patentansprüche

1. Prozessor medizinischer Vorrichtungen (10), umfassend:
ein Becken (14) mit einer Öffnung (80) zum Aufnehmen einer medizinischen Vorrichtung darin für Verarbeitung und einem Rand (82) am Umfang der Öffnung (80);
einen Deckelrahmen (90); und
eine Dichtungsfensteranordnung (100), die abnehmbar an dem Deckelrahmen (90) montiert ist;
wobei die Dichtungsfensteranordnung (100) ein Fenster (102) und eine an dem Fenster (102) befestigte elastomere Dichtung (104) beinhaltet;
wobei der Deckelrahmen (90) mit der daran montierten Dichtungsfensteranordnung (100) relativ zu dem Rand (82) des Beckens (14) zwischen einer offenen Position und einer abgedichteten Position verschiebbar ist, wobei sich die Dichtungsfensteranordnung (100) in der offenen Position in einem Winkel ungleich Null relativ zu der Dichtungsfensteranordnung (100) in der abgedichteten Position befindet und die Dichtungsfensteranordnung (100) die Öffnung (80) freilegt und wobei die elastomere Dichtung (104) der Dichtungsfensteranordnung (100) in der abgedichteten Position in Dichtungskontakt mit dem Rand (82) des Beckens (14) steht, um eine Dichtung zwischen dem Fenster (102) und dem Rand (82) zu erzeugen, um die Öffnung (80) des Beckens (14) von der Außenseite des Prozessors medizinischer Vorrichtungen (10) abzudichten; und
**dadurch gekennzeichnet, dass**
der Deckelrahmen (90) eine Schienenkonstruktion (120) beinhaltet, die konfiguriert ist, um eine Kantenkonstruktion (130) des Fensters (102) der Dichtungsfensteranordnung (100) gleitend zu führen, um die Dichtungsfensteranordnung (100) abnehmbar an dem Deckelrahmen (90) zu montieren.

2. Prozessor medizinischer Vorrichtungen (10) nach Anspruch 1, wobei die Schienenkonstruktion (120) mit daran gesicherten Polsterstreifen (190) ausgekleidet ist, gegen die eine Oberfläche (202) der Kantenkonstruktion (130) des Fensters (102) während gleitender Bewegung relativ zu der Schienenkonstruktion (120) gleitet.

3. Prozessor medizinischer Vorrichtungen (10) nach Anspruch 2, wobei die Polsterstreifen (190) eines oder mehrere von Filzstreifen und Glasfaser beinhalten.

4. Prozessor medizinischer Vorrichtungen (10) nach einem der Ansprüche 1-3, wobei die Schienenkonstruktion (120) mindestens eine Stützschiene beinhaltet und die Kantenkonstruktion (130) mindestens eine Kante beinhaltet.

5. Prozessor medizinischer Vorrichtungen (10) nach einem der Ansprüche 1-4, wobei die Schienenkonstruktion (120) linke, rechte und hintere Stützschienen (122, 124, 126) beinhaltet und die Kantenkonstruktion (130) linke, rechte und hintere Kanten (132, 134, 136) beinhaltet und der Deckelrahmen (90) ferner ein offenes vorderes Ende (150) beinhaltet, das derart konfiguriert ist, dass die linken, rechten und hinteren Kanten (132, 134, 136) durch das offene vordere Ende (150) einführbar und entfernbar sind und durch die jeweiligen linken, rechten und hinteren Stützschienen (122, 124, 126) gleitend geführt werden.

6. Prozessor medizinischer Vorrichtungen (10) nach einem der Ansprüche 1-5, wobei die Schienenkonstruktion (120) eine konkave Konstruktion aufweist und die Kantenkonstruktion (130) des Fensters (102) eine konvexe Konstruktion aufweist, derart, dass die Kantenkonstruktion (130) des Fensters (102) innerhalb der konkaven Konstruktion der Schienenkonstruktion (120) gleitend bewegbar ist.

7. Prozessor medizinischer Vorrichtungen (10) nach Anspruch 6, wobei die konkave Konstruktion im Querschnitt C-förmig ist und Polsterstreifen (190) an einer Unterseite (192) eines der Arme der C-förmigen Konstruktion gesichert sind, gegen die eine Oberfläche (202) der konvexen Konstruktion des Fensters (102) während gleitender Bewegung innerhalb der konkaven Konstruktion gleitet.

8. Prozessor medizinischer Vorrichtungen (10) nach einem der Ansprüche 1-7, wobei der Deckelrahmen (90) an dem Becken (14) für schwenkbare Bewegung um eine Scharnierachse (210) montiert ist und die Dichtungsfensteranordnung (100) abnehmbar an dem Deckelrahmen (90) für gleitende Bewegung in einer Richtung senkrecht zu der Scharnierachse (210) montiert ist.

9. Prozessor medizinischer Vorrichtungen (10) nach einem der Ansprüche 1-8, wobei der Deckelrahmen (90) ferner ein offenes vorderes Ende (150) beinhaltet, das derart konfiguriert ist, dass die Kantenkonstruktion (130) des Fensters (102) durch das offene vordere Ende (150) einführbar und entfernbar ist und durch die Schienenkonstruktion (120) gleitend geführt wird.

10. Prozessor medizinischer Vorrichtungen (10) nach einem der Ansprüche 1-9, ferner umfassend eine Abdeckplatte (230), die abnehmbar an einem vorderen Stützelement (160) des Deckelrahmens (90) montiert ist, um eine vordere Kante (240) der Kantenkonstruktion (130) des Fensters (102) zu stützen und eine gleitende Bewegung des Fensters (102) relativ zu der Schienenkonstruktion (120) zu verhindern.

11. Prozessor medizinischer Vorrichtungen (10) nach einem der Ansprüche 1-10, wobei der Rand (82), der Deckelrahmen (90) und die elastomere Dichtung (104) jeweils ringförmig sind.

12. Prozessor medizinischer Vorrichtungen (10) nach einem der Ansprüche 1-11, wobei die Dichtungsfensteranordnung (100) derart konfiguriert ist, dass, wenn sich der Deckelrahmen (90) in der abgedichteten Position befindet, das Fenster (102) ein Betrachten des Inneren des Beckens (14) ermöglicht.

## Revendications

1. Processeur de dispositif médical (10), comprenant :
un bassin (14) ayant une ouverture (80) pour recevoir en son sein un dispositif médical à traiter et un rebord (82) au niveau du périmètre de l'ouverture (80) ;
un cadre de couvercle (90) ; et,
un ensemble fenêtre d'étanchéité (100) monté de manière amovible sur le cadre de couvercle (90) ;
dans lequel l'ensemble fenêtre d'étanchéité (100) comporte une fenêtre (102) et un joint élastomère (104) fixé à la fenêtre (102) ;
dans lequel le cadre de couvercle (90) avec l'ensemble fenêtre d'étanchéité (100) monté sur celui-ci est déplaçable par rapport au rebord (82) du bassin (14) entre une position ouverte et une position scellée, dans lequel, dans la position ouverte, l'ensemble fenêtre d'étanchéité (100) est à un angle non nul par rapport à l'ensemble fenêtre d'étanchéité (100) dans la position scellée et l'ensemble fenêtre d'étanchéité (100) expose l'ouverture (80), et dans lequel, dans la position scellée, le joint élastomère (104) de l'ensemble fenêtre d'étanchéité (100) est en contact d'étanchéité avec le rebord (82) du bassin (14) pour créer un joint entre la fenêtre (102) et le rebord (82) afin de sceller l'ouverture (80) du bassin (14) de l'extérieur du processeur de dispositif médical (10) ; et
**caractérisé en ce que**
le cadre de couvercle (90) comporte une structure de rail (120) configurée pour guider de manière coulissante une structure de bord (130) de la fenêtre (102) de l'ensemble fenêtre d'étanchéité (100) afin de monter de manière amovible l'ensemble fenêtre d'étanchéité (100) sur le cadre de couvercle (90).

2. Processeur de dispositif médical (10) selon la revendication 1, dans lequel la structure de rail (120) est revêtue de bandes d'amortissement (190) fixées à celle-ci contre lesquelles coulisse une surface (202) de la structure de bord (130) de la fenêtre (102) lors d'un mouvement de coulissement par rapport à la structure de rail (120).

3. Processeur de dispositif médical (10) selon la revendication 2, dans lequel les bandes d'amortissement (190) comportent une ou plusieurs parmi des bandes de feutre et de la fibre de verre.

4. Processeur de dispositif médical (10) selon l'une quelconque des revendications 1 à 3, dans lequel la structure de rail (120) comporte au moins un rail de support et la structure de bord (130) comporte au moins un bord.

5. Processeur de dispositif médical (10) selon l'une quelconque des revendications 1 à 4, dans lequel la structure de rail (120) comporte des rails de support gauche, droit et arrière (122, 124, 126), et la structure de bord (130) comporte des bords gauche, droit et arrière (132, 134, 136), et le cadre de couvercle (90) comporte en outre une extrémité avant ouverte (150) qui est configurée de sorte que les bords gauche, droit et arrière (132, 134, 136) sont insérables et amovibles à travers l'extrémité avant ouverte (150) et guidés de manière coulissante par les rails de support gauche, droit et arrière respectifs (122, 124, 126).

6. Processeur de dispositif médical (10) selon l'une quelconque des revendications 1 à 5, dans lequel la structure de rail (120) a une structure concave et la structure de bord (130) de la fenêtre (102) a une structure convexe de sorte que la structure de bord (130) de la fenêtre (102) peut se déplacer de manière coulissante à l'intérieur de la structure concave de la structure de rail (120).

7. Processeur de dispositif médical (10) selon la revendication 6, dans lequel la structure concave a une section transversale en forme de C et des bandes d'amortissement (190) sont fixées à une face inférieure (192) de l'un des bras de la structure en forme de C contre laquelle coulisse une surface (202) de la structure convexe de la fenêtre (102) lors d'un mouvement de coulissement à l'intérieur de la structure concave.

8. Processeur de dispositif médical (10) selon l'une quelconque des revendications 1 à 7, dans lequel le cadre de couvercle (90) est monté sur le bassin (14) pour un mouvement pivotant autour d'un axe de charnière (210) et l'ensemble fenêtre d'étanchéité (100) est monté de manière amovible sur le cadre de couvercle (90) pour un mouvement coulissant dans une direction perpendiculaire à l'axe de charnière (210).

9. Processeur de dispositif médical (10) selon l'une quelconque des revendications 1 à 8, dans lequel le cadre de couvercle (90) comporte en outre une extrémité avant ouverte (150) qui est configurée de sorte que la structure de bord (130) de la fenêtre (102) est insérable et amovible à travers l'extrémité avant ouverte (150) et guidée de manière coulissante par la structure de rail (120).

10. Processeur de dispositif médical (10) selon l'une quelconque des revendications 1 à 9, comprenant en outre un panneau protecteur (230) monté de manière amovible sur un élément de support avant (160) du cadre de couvercle (90) pour supporter un bord avant (240) de la structure de bord (130) de la fenêtre (102) et empêcher un mouvement de coulissement de la fenêtre (102) par rapport à la structure de rail (120).

11. Processeur de dispositif médical (10) selon l'une quelconque des revendications 1 à 10, dans lequel chacun du rebord (82), du cadre de couvercle (90) et du joint élastomère (104) est de forme annulaire.

12. Processeur de dispositif médical (10) selon l'une quelconque des revendications 1 à 11, dans lequel l'ensemble fenêtre d'étanchéité (100) est configuré de sorte que, lorsque le cadre de couvercle (90) est dans la position scellée, la fenêtre (102) permet de visualiser l'intérieur du bassin (14).
